# EUROPÄISCHE PATENTSCHRIFT

(11) **EP 2 926 385 B1**
(45) Veröffentlichungstag und Bekanntmachung des Hinweises auf die Patenterteilung: **05.08.2020**
(21) Anmeldenummer: 13786424.5
(22) Anmeldetag: 04.11.2013
(51) Int. Cl.: H01L 51/00, C07D 495/00, C07D 471/04, C09B 57/00, C09B 11/28, C09B 15/00, C09B 17/00, C09B 19/00, C09B 21/00

(54) **ELEKTRONISCHE VORRICHTUNG**
ELECTRONIC DEVICE
DISPOSITIF ÉLECTRONIQUE

(30) Priorität: 30.11.2012 EP 12008039
(43) Veröffentlichungstag der Anmeldung: 07.10.2015
(73) Patentinhaber: Merck Patent GmbH, 64293 Darmstadt (DE)
(72) Erfinder: PAN, Junyou, 60320 Frankfurt am Main (DE); PARHAM, Amir Hossain, 60486 Frankfurt am Main (DE); SCHEURICH, René, Peter, 64846 Gross-Zimmern (DE); RUDOLPH, Thomas, 64291 Darmstadt (DE)
(86) Internationale Anmeldenummer: PCT/EP2013/003309
(87) Internationale Veröffentlichungsnummer: WO 2014/082705

(56) Entgegenhaltungen:
- WO-A1-2011/126225
- US-B1- 6 333 146
- M. G. MAISURADZE ET AL.: "Synthesis of novel heterocyclic systems, nemzo[b]furobenzimidazoles", CHEMISTRY OF HETEROCYCLIC COMPOUNDS, Bd. 48, Nr. 7, Oktober 2012 (2012-10), Seiten 1125-1126, XP002695063,

## Beschreibung

Die vorliegende Anmeldung betrifft eine elektronische Vorrichtung, welche mindestens eine organische Schicht enthält, welche wiederum mindestens eine Verbindung der Formel (I), wie unten definiert, enthält. Die Verbindung der Formel (I) stellt ein Funktionsmaterial dar, welches bevorzugt als Elektronentransportmaterial und/oder als Matrixmaterial für Emitterverbindungen in der elektronischen Vorrichtung eingesetzt wird. Die Verbindung der Formel (I-1) eignet sich besonders zur Verwendung in einer elektronischen Vorrichtung, insbesondere in den oben genannten Funktionen.

Unter dem Begriff elektronische Vorrichtung werden gemäß der vorliegenden Erfindung allgemein elektronische Vorrichtungen verstanden, welche organische Materialien enthalten. Solche Vorrichtungen werden auch als organische elektronische Vorrichtungen bezeichnet. Unter diesem Begriff werden im Rahmen der vorliegenden Anmeldung bevorzugt OLEDs verstanden sowie einige weitere Ausführungsformen von organischen elektronischen Vorrichtungen, die in der Anmeldung an späterer Stelle offenbart werden.

Der allgemeine Aufbau sowie das Funktionsprinzip von OLEDs ist dem Fachmann bekannt und unter anderem in US 4539507, US 5151629, EP 0676461 und WO 1998/27136 beschrieben.

Betreffend die Leistungsdaten von elektronischen Vorrichtungen sind weitere Verbesserungen erforderlich, insbesondere in Hinblick auf eine breite kommerzielle Verwendung, beispielsweise in Displays oder als Lichtquellen. Von besonderer Bedeutung sind in diesem Zusammenhang die Lebensdauer, die Effizienz und die Betriebsspannung der elektronischen Vorrichtungen sowie die realisierten Farbwerte bei Lichtemission.

Ein Ansatzpunkt zur Verbesserung dieser Parameter ist die Auswahl von geeigneten organischen Materialien zur Verwendung in den elektronischen Vorrichtungen. Durch Auswahl von geeigneten Materialien können Leistungsdaten der elektronischen Vorrichtung entscheidend beeinflusst werden.

Zur Verwendung als elektronentransportierende Verbindungen, beispielsweise in der Elektronentransportschicht oder als Matrixmaterial in der emittierenden Schicht, sind im Stand der Technik eine Vielzahl von Materialien bekannt.

Dazu zählen beispielsweise LiQ, AIQ₃ und andere Chinolinate (US 4356429, US 2007/0092753), sowie heteroaromatische Verbindungen, z. B. Triazine (WO 2010/015306, WO 2007/063754 oder WO 2008/056746), Carbazole (WO 2005/039246, US 2005/0069729, JP 2004/288381, EP 1205527 oder WO 2008/086851), sowie Ketone, Phosphinoxide, Sulfoxide und Sulfone (WO 2004/093207, WO 2010/006680 oder WO 2005/003253).

Weiterhin im Stand der Technik bekannt ist die Verwendung von Benzimidazol-Derivaten und ähnlichen Heteroaromaten als Elektronentransportmaterialien und/oder als Matrixmaterialien in der emittierenden Schicht von elektronischen Vorrichtungen (WO 2004/080975, US 2009/184633, JP 2008/130754 und JP 2006/156847).

Elektronische Vorrichtungen enthaltend die Verbindungen weisen jedoch Verbesserungspotential auf, insbesondere bezüglich Effizienz, Betriebsspannung, Lebensdauer und Prozessierbarkeit. Von besonderer Bedeutung sind dabei Lebensdauer und Betriebsspannung der elektronischen Vorrichtung.

Weiterhin im Stand der Technik bekannt sind OLEDs enthaltend Benzimidazol-Derivate, bei denen zwei Benzimidazol-Gruppen symmetrisch über einen zentralen Fünfring miteinander verbunden sind (WO 2011/126225). Die Verbindungen werden in der Elektronentransportschicht oder in der emittierenden Schicht als Matrixmaterialien eingesetzt.

Es besteht also weiterhin Interesse an elektronischen Vorrichtungen, insbesondere OLEDs, mit hoher Effizienz und niedriger Betriebsspannung sowie langer Lebensdauer.

Überraschend wurde nun gefunden, dass elektronische Vorrichtungen enthaltend ein Benzimidazol-Derivat, welches einen ankondensierten Heterocyclus mit einem wiederum ankondensierten aromatischen Sechsring trägt, die oben genannten Eigenschaften aufweisen und somit die technische Aufgabe lösen. Benzimidazol-Derivate sind im Stand der Technik bekannt (z.B in Maisuradze et al., Chemistry of Heterocyclic Compounds, Bd.48, Nr.7, 2012, pages 1125-1126 oder in US 6,333,146).

Gegenstand der vorliegenden Erfindung ist somit eine elektronische Vorrichtung, enthaltend Anode, Kathode und mindestens eine organische Schicht enthaltend mindestens eine Verbindung der Formel (I) 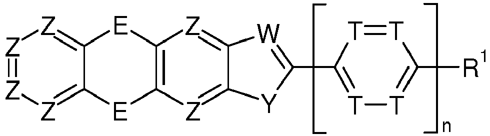 Formel (I)
wobei gilt:
- E: ist bei jedem Auftreten gleich oder verschieden gewählt aus einer Einfachbindung, B(R¹), C=O, N(R¹), P(R¹), P(=O)R¹, O, S, S=O und S(=O)₂, wobei nicht beide Gruppen E eine Einfachbindung sein dürfen;
- T: ist bei jedem Auftreten gleich oder verschieden CR¹ oder N;
- W: ist CR¹ oder N;
- Y: ist N(R¹), O oder S;
- Z: ist bei jedem Auftreten gleich oder verschieden CR² oder N;
- R¹: ist bei jedem Auftreten gleich oder verschieden H, D, F, C(=O)R³, CN, Si(R³)₃, N(R³)₂, P(=O)(R³)₂, S(=O)R³, S(=O)₂R³, eine geradkettige Alkyl-, oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F oder CN ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann;
- R²: ist bei jedem Auftreten gleich oder verschieden H, D, F, C(=O)R³, CN, Si(R³)₃, N(R³)₂, P(=O)(R³)₂, S(=O)R³, S(=O)₂R³, eine geradkettige Alkyl-, oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F oder CN ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann, wobei zwei oder mehr Reste R² miteinander verknüpft sein können und einen aliphatischen oder heteroaliphatischen Ring bilden können;
- R³: ist bei jedem Auftreten gleich oder verschieden H, D, F, C(=O)R⁴, CN, Si(R⁴)₃, N(R⁴)₂, P(=O)(R⁴)₂, S(=O)R⁴, S(=O)₂R⁴, eine geradkettige Alkyl-, oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können und wobei ein oder mehrere H-Atome in den oben genannten Gruppen durch D, F oder CN ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxy- oder Heteroaryloxygruppe mit 5 bis 30 aromatischen Ringatomen, die durch einen oder mehrere Reste R⁴ substituiert sein kann, wobei zwei oder mehr Reste R³ miteinander verknüpft sein können und einen Ring bilden können;
- R⁴: ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden;
- n: ist gleich 0 oder 1;
wobei mindestens eine Gruppe R¹ oder R² in der Verbindung der Formel (I) gewählt ist aus einem aromatischen oder heteroaromatischen Ringsystem mit 5 bis 40 aromatischen Ringatomen, das mit einem oder mehreren Resten R³ substituiert sein kann.

Die erfindungsgemäßen Verbindungen weisen bevorzugt einen oder mehrere der unten angegebenen Vorteile auf:
- Bei Verwendung der erfindungsgemäßen Verbindungen in elektronischen Vorrichtungen können eine hohe Leistungseffizienz und eine niedrige Betriebsspannung der Vorrichtungen erreicht werden.
- Bei Verwendung der erfindungsgemäßen Verbindungen wird eine hohe Stabilität bzw. eine lange Lebensdauer der elektronischen Vorrichtung erhalten.
- Die erfindungsgemäßen Verbindungen weisen deutlich bessere Löslichkeit und Filmbildungseigenschaften auf im Vergleich zu im Stand der Technik bekannten Materialien, insbesondere im Vergleich zu Matrixmaterialien mit Carbazol-Grundkörper.

Es folgen allgemeine Definitionen für chemische Gruppen:
Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

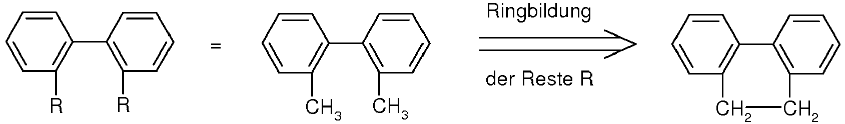

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

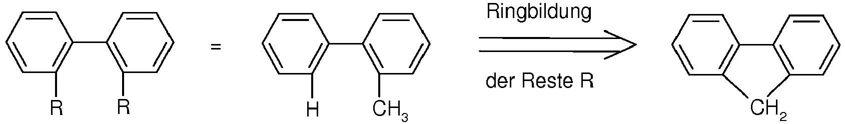

Gemäß einer bevorzugten Ausführungsform ist mindestens eine Gruppe R¹ in der Verbindung der Formel (I) gewählt aus einem aromatischen oder heteroaromatischen Ringsystem mit 5 bis 40 aromatischen Ringatomen, das mit einem oder mehreren Resten R³ substituiert sein kann.

Gemäß einer besonders bevorzugten Ausführungsform ist mindestens eine Gruppe R¹ oder R², bevorzugt mindestens eine Gruppe R¹, in der Verbindung der Formel (I) gewählt aus einer Gruppe enthaltend mindestens eine der folgenden Gruppen:
- Heteroarylgruppen mit 5 bis 20 aromatischen Ringatomen, die mindestens einen heteroaromatischen Fünfring mit zwei oder mehr Heteroatomen gewählt aus N, O und S enthalten;
- Heteroarylgruppen mit 6 bis 20 aromatischen Ringatomen, die mindestens einen heteroaromatischen Sechsring mit einem oder mehreren Heteroatomen gewählt aus N, O und S enthalten; und
- Carbazolgruppen.

Dabei werden unter Carbazolgruppen auch Carbazolderivate mit ankondensierten Gruppen, wie beispielsweise Indenocarbazole oder Indolocarbazole, sowie Carbazolderivate, in denen ein oder mehrere Kohlenstoffatome in den aromatischen Sechsringen durch Stickstoff ersetzt sind, verstanden.

Weiterhin bevorzugt ist mindestens eine Gruppe R¹ oder R² in der Verbindung der Formel (I), besonders bevorzugt mindestens eine Gruppe R¹, gewählt aus Gruppen der Formeln (Het-a) bis (Het-e) 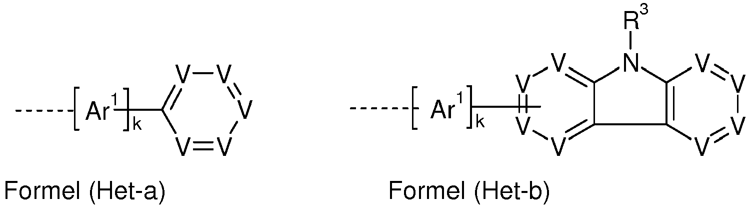 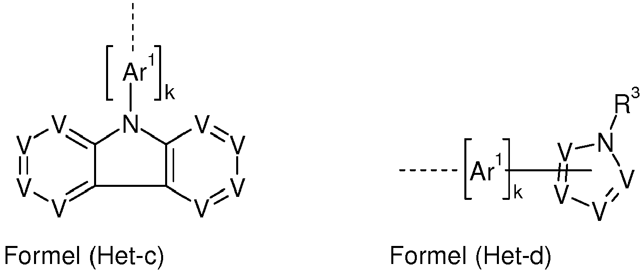 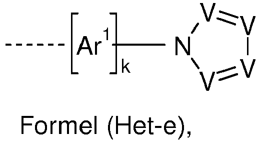 wobei
- Ar¹: ein aromatisches oder heteroaromatisches Ringsystem mit 5 bis 20 aromatischen Ringatomen ist, das mit einem oder mehreren Resten R³ substituiert sein kann;
- V: bei jedem Auftreten gleich oder verschieden N oder CR³ ist;
- k: gleich 0 oder 1 ist;
und die gestrichelte Linie die Bindung an den Rest der Verbindung bezeichnet;
und wobei in Formel (Het-a), (Het-d) und (Het-e) mindestens eine Gruppe V im Ring gleich N ist.

Gemäß einer bevorzugten Ausführungsform hat die Verbindung der Formel (I) ein Molekulargewicht von 250 bis 1500 Da, besonders bevorzugt 275 bis 1250 Da und ganz besonders bevorzugt 300 bis 1000 Da.

Gemäß einer weiteren bevorzugten Ausführungsform ist die Verbindung der Formel (I) asymmetrisch, besonders bevorzugt asymmetrisch in der Art, dass sie asymmetrisch bezüglich jeder beliebigen, auf der Papierebene senkrecht stehenden Spiegelebene ist. Dies hat den Vorteil, dass die Verbindung eine bessere Löslichkeit aufweist. Weiterhin und bevorzugt in Kombination mit dem oben genannten Vorteil hat dies den Vorteil, dass die Verbindungen ein höheres T1-Niveau aufweisen.

Gemäß einer bevorzugten Ausführungsform ist n gleich 1.

Bevorzugt ist weiterhin, dass nicht mehr als drei Gruppen T in der Formel (I) gleich N sind. Weiterhin bevorzugt sind nicht mehr als zwei benachbarte Gruppen T gleich N. Besonders bevorzugt sind alle Gruppen T gleich CR¹.

Bevorzugt ist weiterhin, dass nicht mehr als drei Gruppen Z pro Ring in der Formel (I) gleich N sind. Weiterhin bevorzugt sind nicht mehr als zwei benachbarte Gruppen Z gleich N. Besonders bevorzugt sind alle Gruppen Z gleich CR².

Bevorzugt ist weiterhin, dass W gleich CR¹ ist.

Bevorzugt ist weiterhin, dass E bei jedem Auftreten gleich oder verschieden eine Einfachbindung, C=O, N(R¹), O, S, S=O oder S(=O)₂ ist, wobei nicht beide Gruppen E eine Einfachbindung sein dürfen.

R¹ ist bevorzugt bei jedem Auftreten gleich oder verschieden H, D, CN, Si(R³)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F oder CN ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann;

R¹ ist besonders bevorzugt bei jedem Auftreten gleich oder verschieden H, D, eine geradkettige Alkylgruppe mit 1 bis 10 C-Atomen oder eine verzweigte oder cyclische Alkylgruppe mit 3 bis 10 C-Atomen, wobei die Alkylgruppen jeweils mit einem oder mehreren Resten R³ substituiert sein können, oder ein aromatisches oder heteroaromatisches Ringsystem mit 6 bis 24 aromatischen Ringatomen, das mit einem oder mehreren Resten R³ substituiert sein kann.

Es ist bevorzugt, dass keine Ringbildung zwischen zwei oder mehr Resten R² vorliegt.

R² ist bevorzugt bei jedem Auftreten gleich oder verschieden H, D, CN, Si(R³)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F oder CN ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann;

R² ist besonders bevorzugt bei jedem Auftreten gleich oder verschieden H, D, eine geradkettige Alkylgruppe mit 1 bis 10 C-Atomen oder eine verzweigte oder cyclische Alkylgruppe mit 3 bis 10 C-Atomen, wobei die Alkylgruppen jeweils mit einem oder mehreren Resten R³ substituiert sein können, oder ein aromatisches oder heteroaromatisches Ringsystem mit 6 bis 24 aromatischen Ringatomen, das mit einem oder mehreren Resten R³ substituiert sein kann.

R³ ist bevorzugt bei jedem Auftreten gleich oder verschieden H, D, CN, Si(R⁴)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können und wobei ein oder mehrere H-Atome in den oben genannten Gruppen durch D, F oder CN ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxy- oder Heteroaryloxygruppe mit 5 bis 30 aromatischen Ringatomen, die durch einen oder mehrere Reste R⁴ substituiert sein kann;

R³ ist besonders bevorzugt bei jedem Auftreten gleich oder verschieden H, D, eine geradkettige Alkylgruppe mit 1 bis 10 C-Atomen oder eine verzweigte oder cyclische Alkylgruppe mit 3 bis 10 C-Atomen, wobei die Alkylgruppen jeweils mit einem oder mehreren Resten R⁴ substituiert sein können, oder ein aromatisches oder heteroaromatisches Ringsystem mit 6 bis 24 aromatischen Ringatomen, das mit einem oder mehreren Resten R⁴ substituiert sein kann.

Bevorzugte Ausführungsformen von Verbindungen der Formel (I) entsprechen den folgenden Formeln (I-A) bis (I-F) 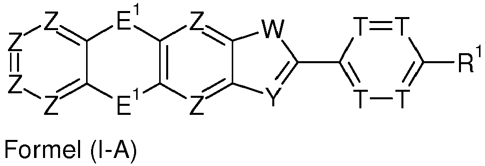 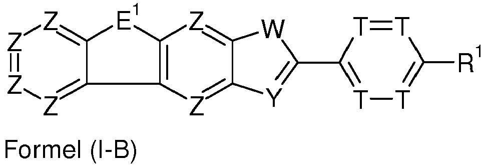 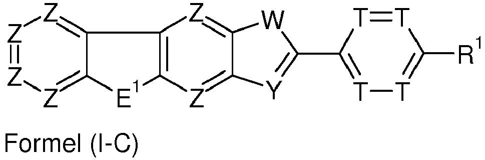 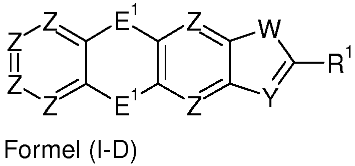 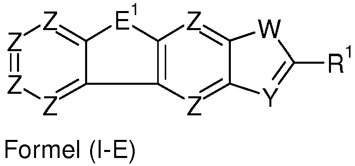 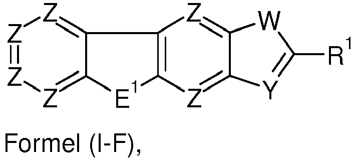 wobei
- E¹: bei jedem Auftreten gleich oder verschieden gewählt ist aus C=O, N(R¹), O und S; und
wobei die restlichen auftretenden Symbole wie oben definiert sind; und
wobei mindestens eine Gruppe R¹ oder R² in der Verbindung der Formeln (I-A) bis (I-F) gewählt ist aus einem aromatischen oder heteroaromatischen Ringsystem mit 5 bis 40 aromatischen Ringatomen, das mit einem oder mehreren Resten R³ substituiert sein kann.

Es gelten die oben angeführten bevorzugten Ausführungsformen der Symbole auch für die Formeln (I-A9 bis (I-F) als bevorzugt.

Insbesondere bevorzugt ist für die Formeln (I-A) bis (I-F) T gleich CR¹.

Weiterhin insbesondere bevorzugt ist für die Formeln (I-A) bis (I-F) Z gleich CR¹.

Weiterhin insbesondere bevorzugt ist für die Formeln (I-A) bis (I-F) W gleich N.

Weiterhin insbesondere bevorzugt ist mindestens eine Gruppe R¹ oder R², bevorzugt mindestens eine Gruppe R¹ in der Verbindung der Formeln (I-A) bis (I-F) gewählt aus einer Gruppe enthaltend mindestens eine der folgenden Gruppen:
- Heteroarylgruppen mit 5 bis 20 aromatischen Ringatomen, die mindestens einen heteroaromatischen Fünfring mit zwei oder mehr Heteroatomen gewählt aus N, O und S enthalten;
- Heteroarylgruppen mit 6 bis 20 aromatischen Ringatomen, die mindestens einen heteroaromatischen Sechsring mit einem oder mehreren Heteroatomen gewählt aus N, O und S enthalten; und
- Carbazolgruppen.

Weiterhin interessant ist eine Verbindung der Formel (I-1), die eine spezielle Ausführungsform der Formel (I) darstellt: 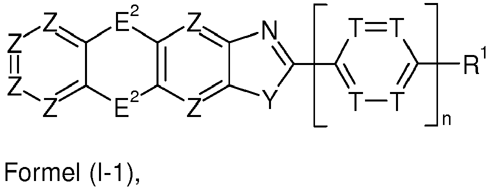 wobei gilt:
- E²: ist bei jedem Auftreten gleich oder verschieden eine Einfachbindung, C=O, N(R¹), O oder S, wobei nicht beide Gruppen E² eine Einfachbindung sein dürfen;
die restlichen Symbole sind definiert wie oben;
und wobei mindestens eine Gruppe R¹ gewählt ist aus Gruppen der Formeln (Het-a) bis (Het-e), wie oben definiert.

Es gelten die oben angeführten bevorzugten Ausführungsformen der Symbole ebenfalls als bevorzugt für Formel (I-1).

Insbesondere bevorzugt ist für Formel (I-1) T gleich CR¹.

Weiterhin insbesondere bevorzugt ist für Formel (I-1) Z gleich CR¹.

Weiterhin insbesondere bevorzugt ist für Formel (I-1) Y gleich NR¹.

Bevorzugt ist für Formel (I-1) E² bei jedem Auftreten gleich oder verschieden eine Einfachbindung, NR¹, O, oder S, wobei nicht beide E² eine Einfachbindung sein können.

Bevorzugte Ausführungsformen von Verbindungen der Formel (I-1) sind die folgenden Verbindungen der Formeln (I-1-A) bis (I-1-F) 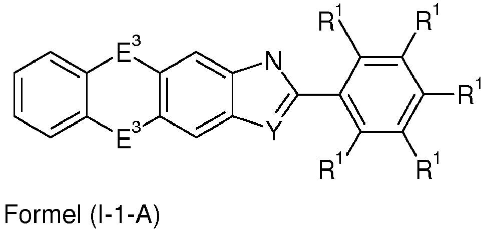 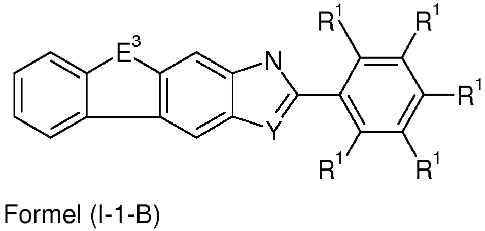 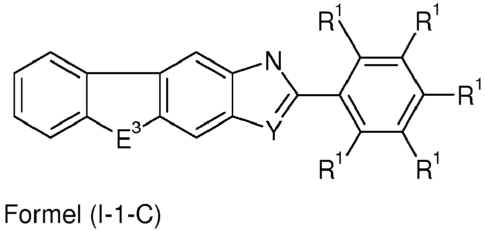 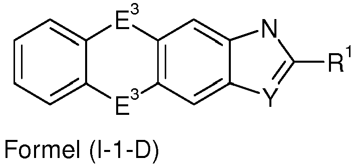 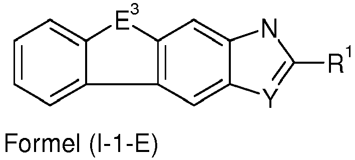 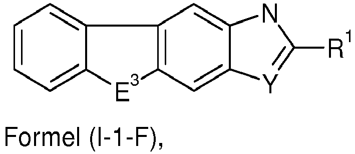 wobei die Verbindungen an allen freien Positionen mit Resten R² substituiert sein können, und wobei
- E³: bei jedem Auftreten gleich oder verschieden C=O, N(R¹), O oder S ist; und
- Y, R¹, R²: wie oben definiert sind; und wobei
mindestens eine Gruppe R¹ gewählt ist aus Gruppen der Formeln (Het-a) bis (Het-e), wie oben definiert.

Es gelten die oben aufgeführten bevorzugten Ausführungsformen von Gruppen ebenfalls als bevorzugt.

Insbesondere gelten die bevorzugten Ausführungsformen von Gruppen R¹ und/oder R² als bevorzugt für Formeln (I-1-A) bis (I-1-F).

Weiterhin insbesondere bevorzugt ist für Formeln (I-1-A) bis (I-1-F) Y gleich NR¹.

Beispiele für Verbindungen gemäß Formel (I) sind in der folgenden Tabelle abgebildet.

| | |
|---|---|
| 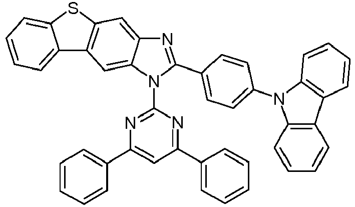 | 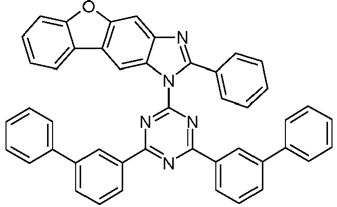 |
| 1 | 2 |
| 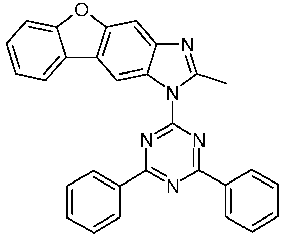 | 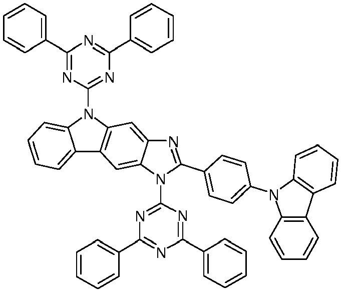 |
| 3 | 4 |
| 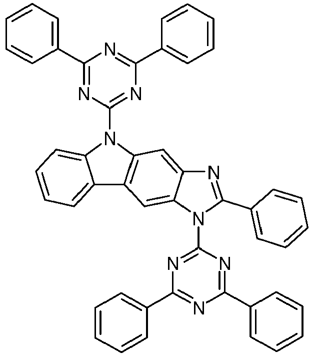 | 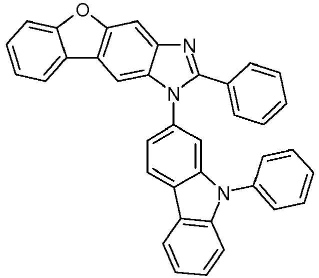 |
| 5 | 6 |
| 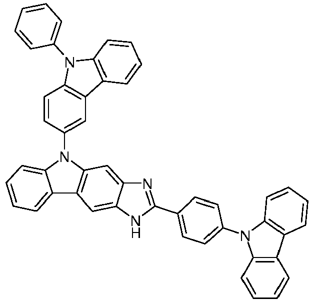 | 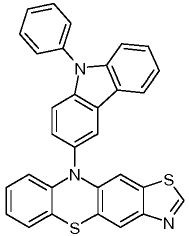 |
| 7 | 8 |
| 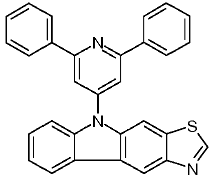 | 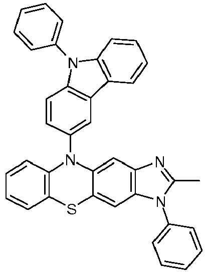 |
| 9 | 10 |
| 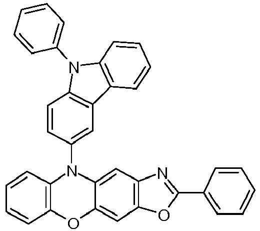 | 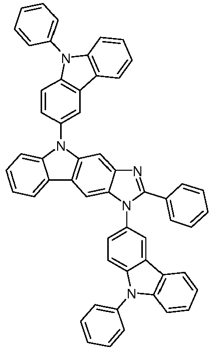 |
| 11 | 12 |
| 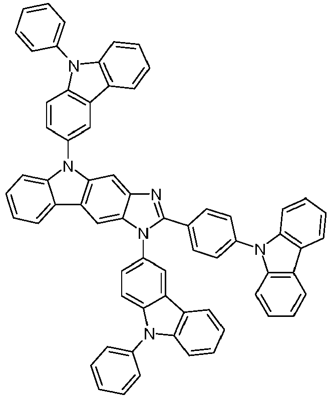 | 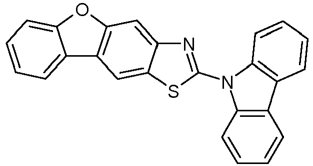 |
| 13 | 14 |
| 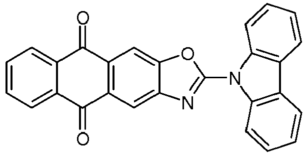 | 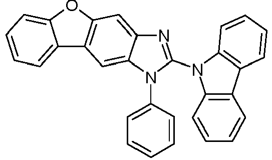 |
| 15 | 16 |
| 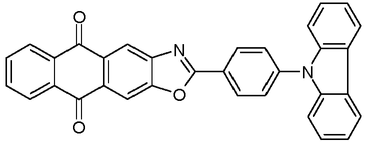 | 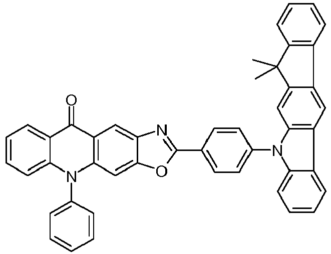 |
| 17 | 18 |
| 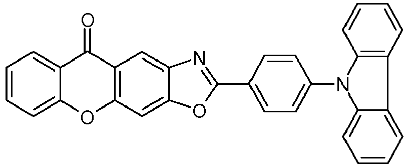 | 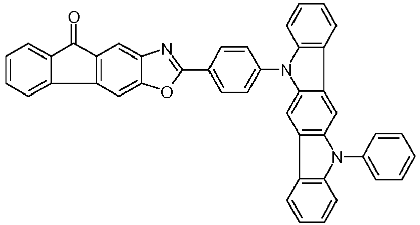 |
| 19 | 20 |
| 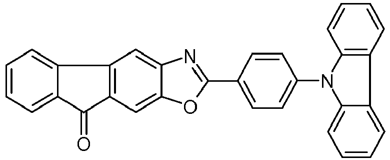 | 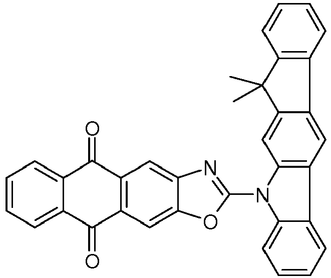 |
| 21 | 22 |
| 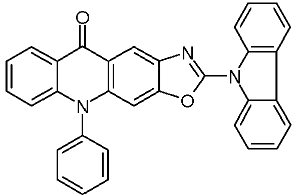 | 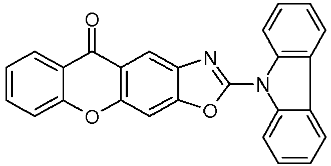 |
| 23 | 24 |
| 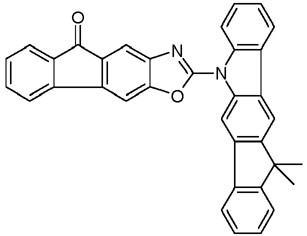 | 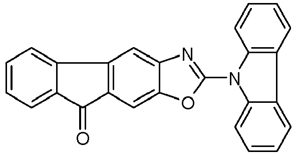 |
| 25 | 26 |
| 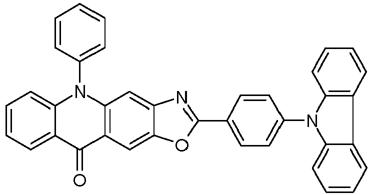 | 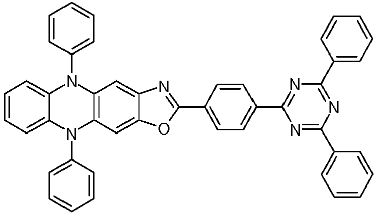 |
| 27 | 28 |
| 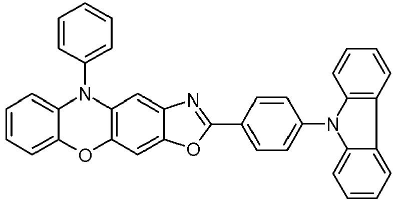 | 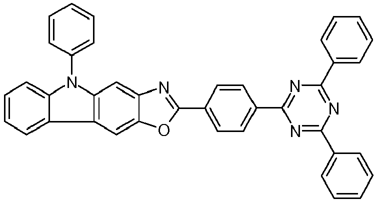 |
| 29 | 30 |
| 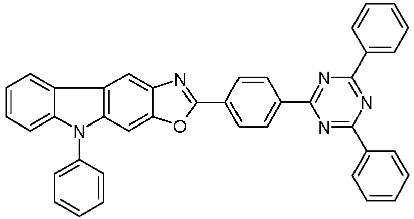 | 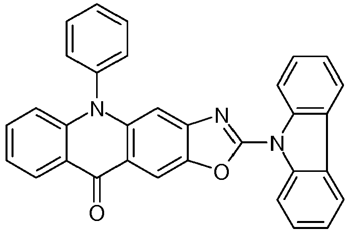 |
| 31 | 32 |
| 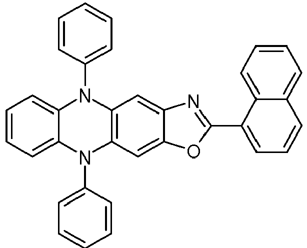 | 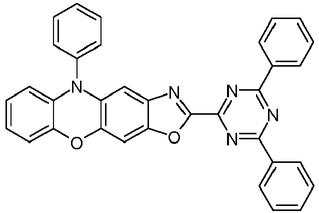 |
| 33 | 34 |
| 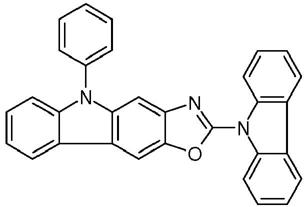 | 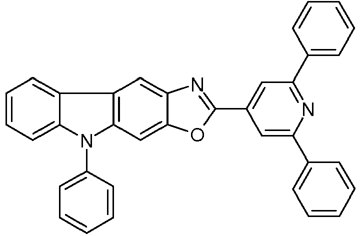 |
| 35 | 36 |
| 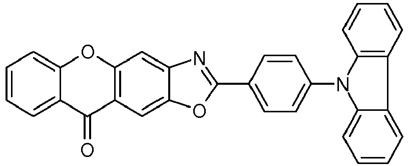 | 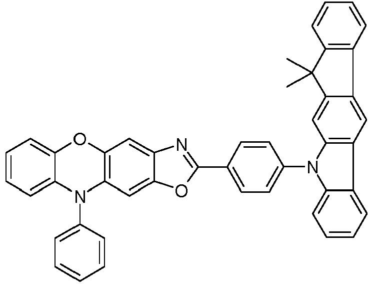 |
| 37 | 38 |
| 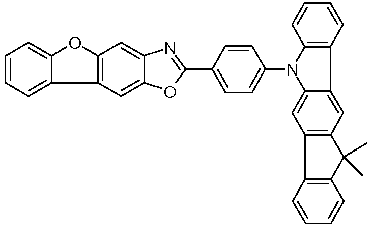 | 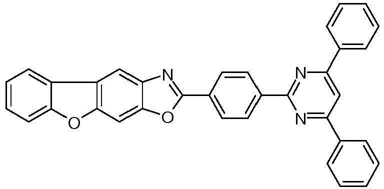 |
| 39 | 40 |
| 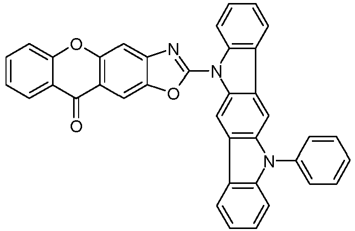 | 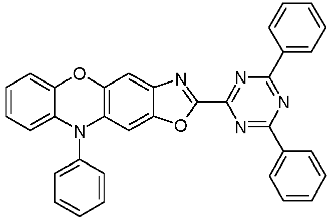 |
| 41 | 42 |
| 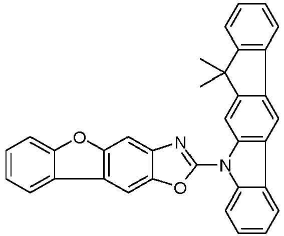 | 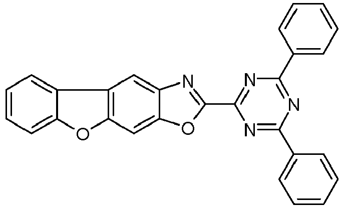 |
| 43 | 44 |
| 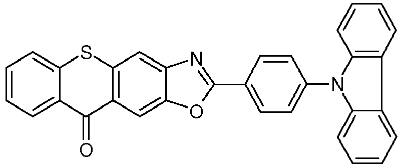 | 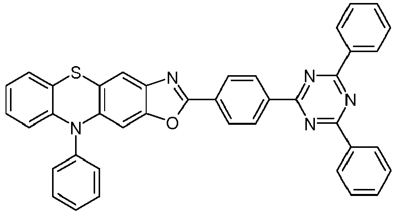 |
| 45 | 46 |
| 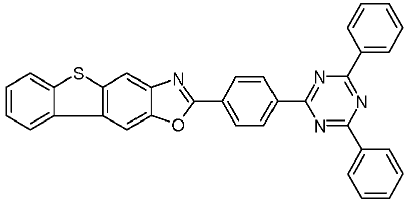 | 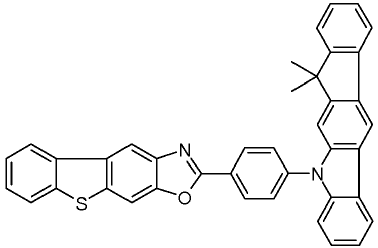 |
| 47 | 48 |
| 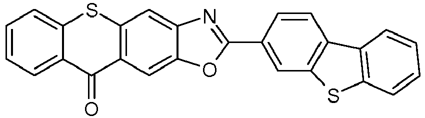 | 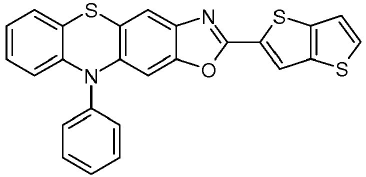 |
| 49 | 50 |
| 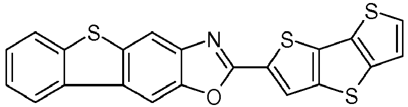 | 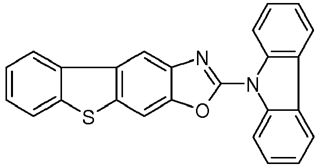 |
| 51 | 52 |
| 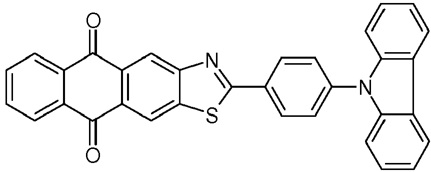 | 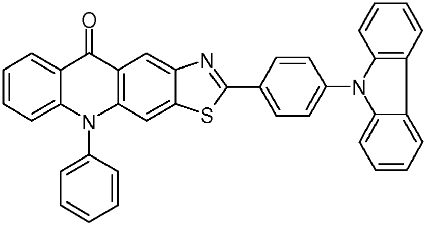 |
| 53 | 54 |
| 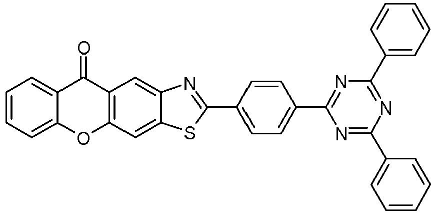 | 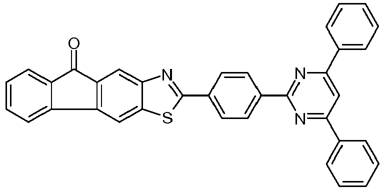 |
| 55 | 56 |
| 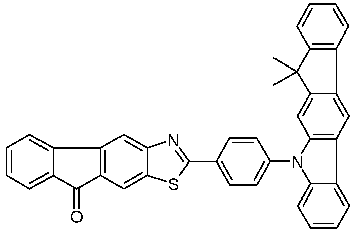 | 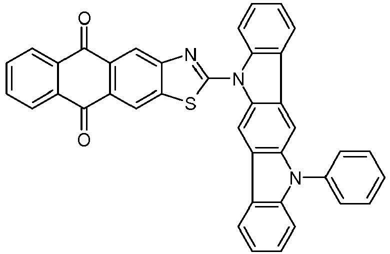 |
| 57 | 58 |
| 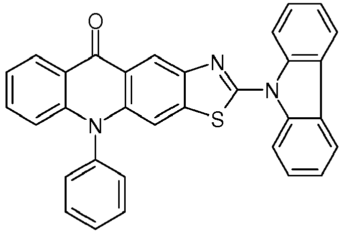 | 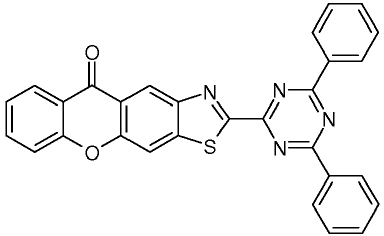 |
| 59 | 60 |
| 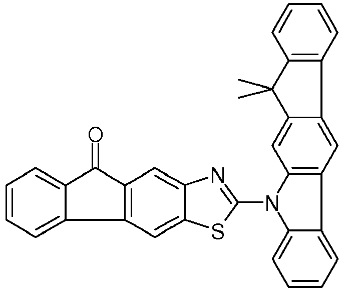 | 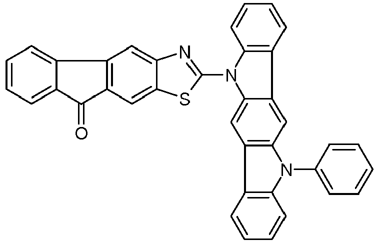 |
| 61 | 62 |
| 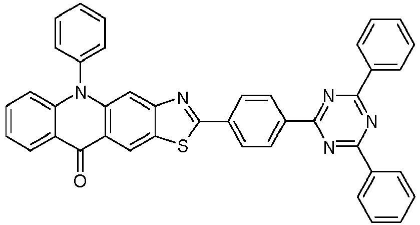 | 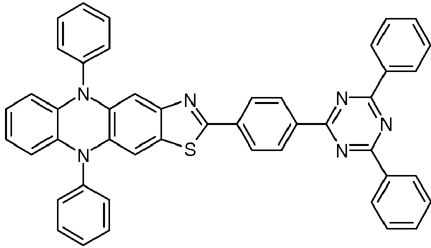 |
| 63 | 64 |
| 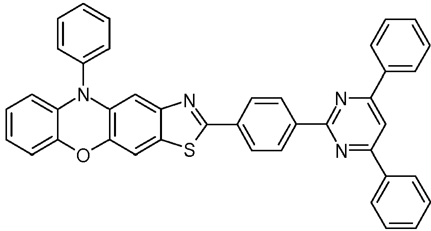 | 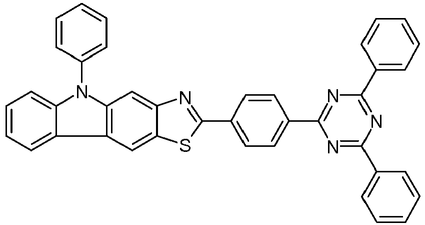 |
| 65 | 66 |
| 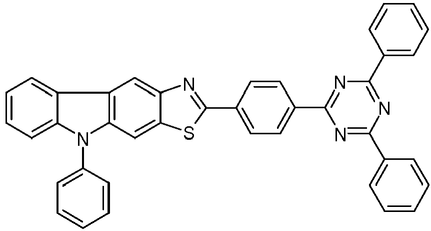 | 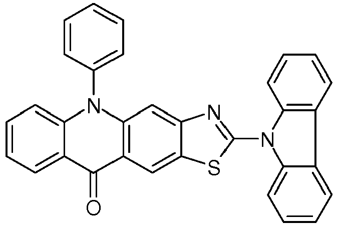 |
| 67 | 68 |
| 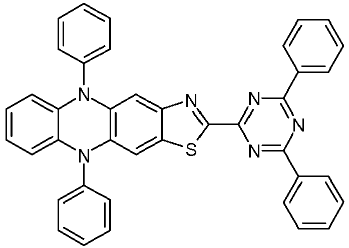 | 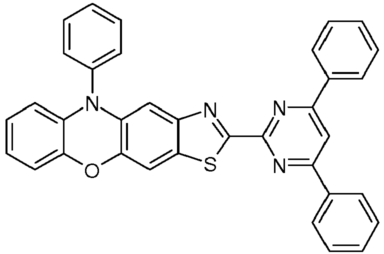 |
| 69 | 70 |
| 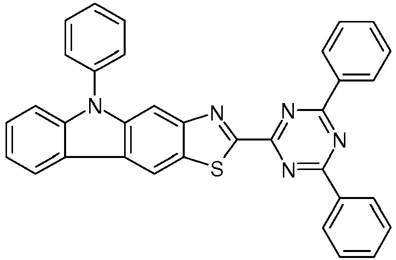 | 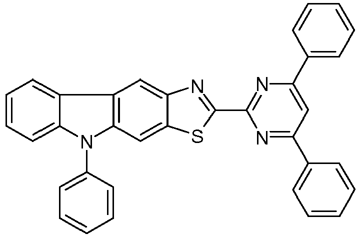 |
| 71 | 72 |
| 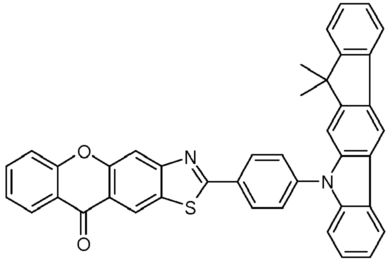 | 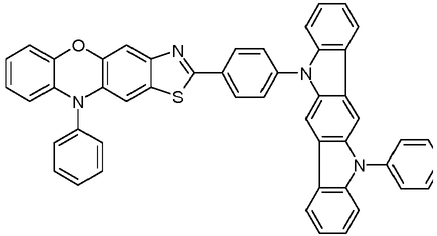 |
| 73 | 74 |
| 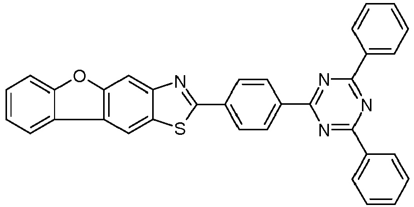 | 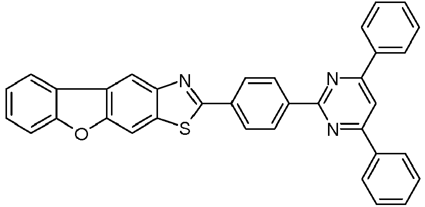 |
| 75 | 76 |
| 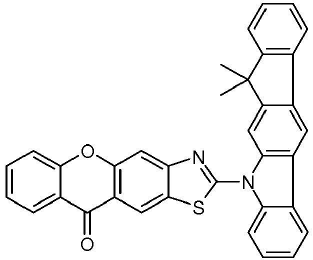 | 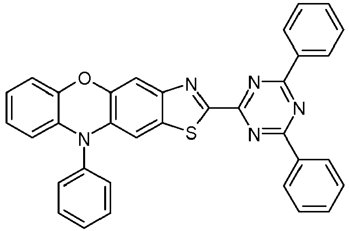 |
| 77 | 78 |
| 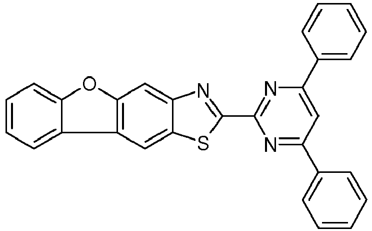 | 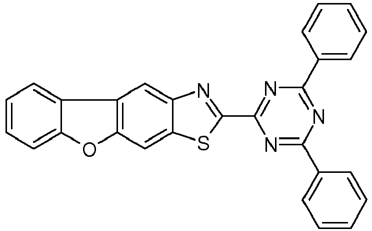 |
| 79 | 80 |
| 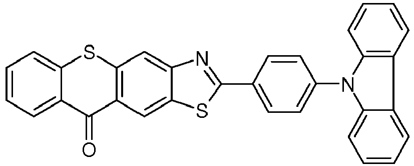 | 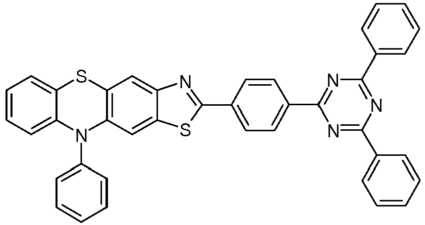 |
| 81 | 82 |
| 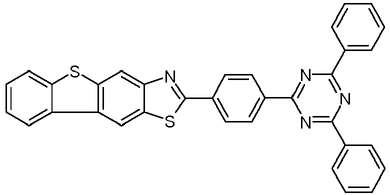 | 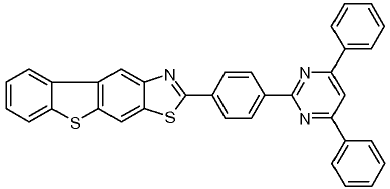 |
| 83 | 84 |
| 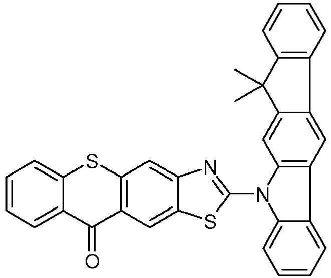 | 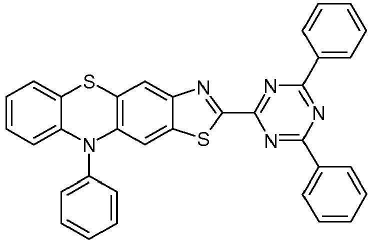 |
| 85 | 86 |
| 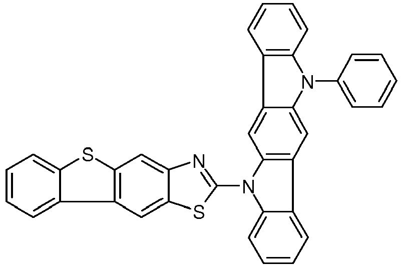 | 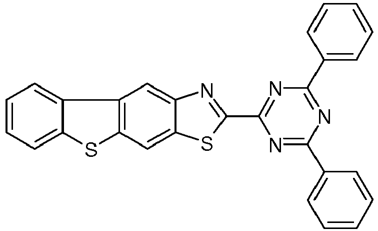 |
| 87 | 88 |
| 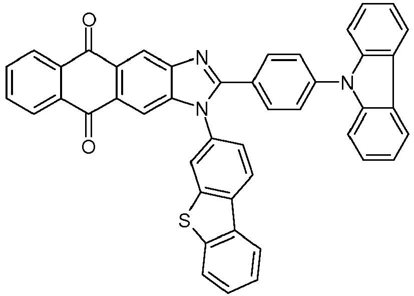 | 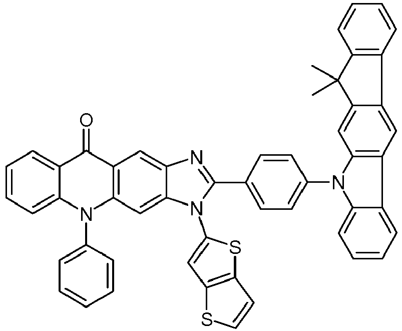 |
| 89 | 90 |
| 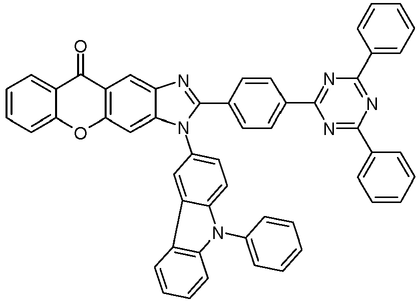 | 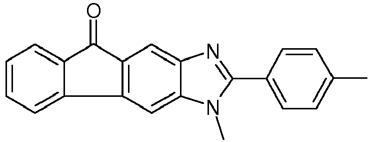 |
| 91 | 92 |
| 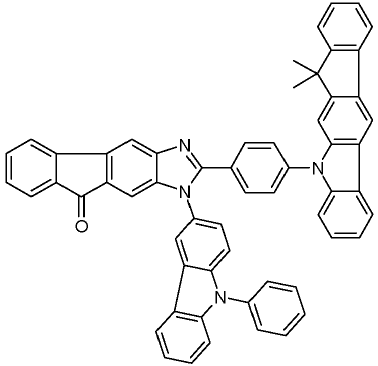 | 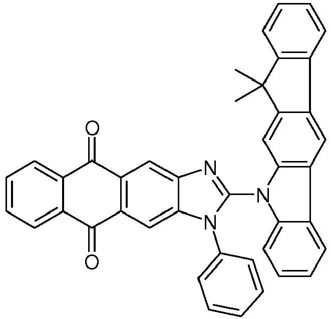 |
| 93 | 94 |
| 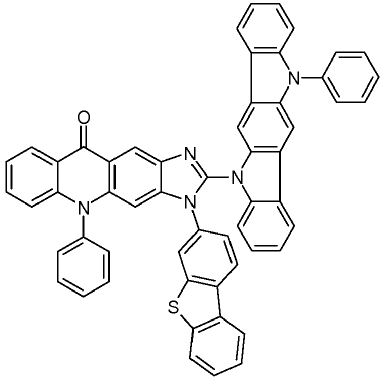 | 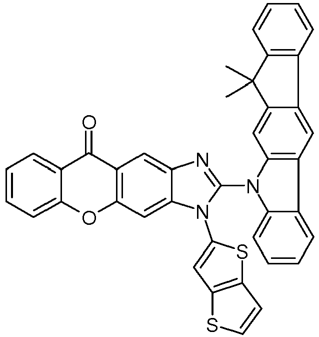 |
| 95 | 96 |
| 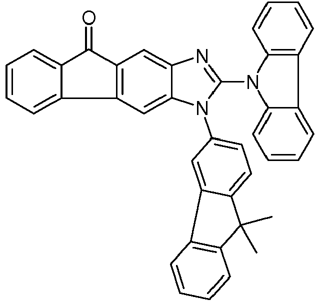 | 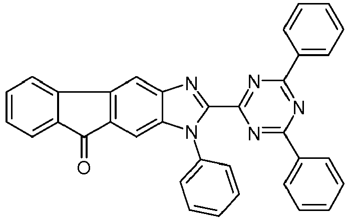 |
| 97 | 98 |
| 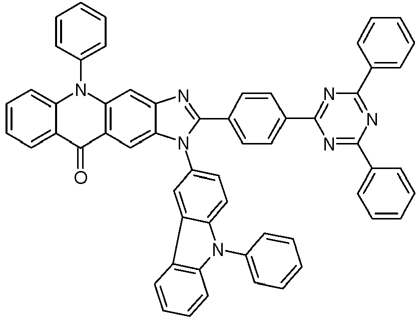 | 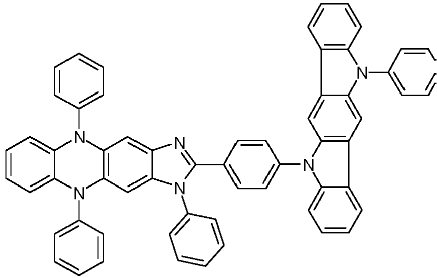 |
| 99 | 100 |
| 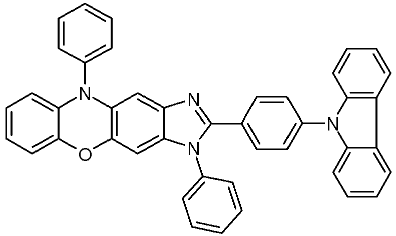 | 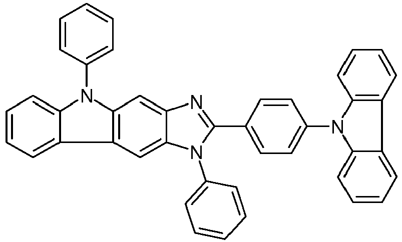 |
| 101 | 102 |
| 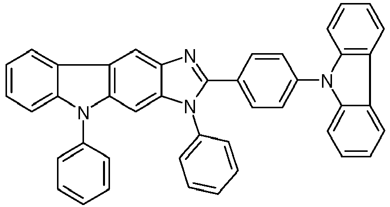 | 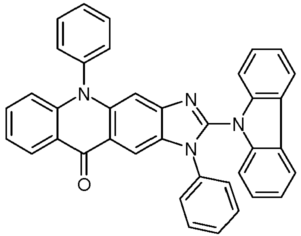 |
| 103 | 104 |
| 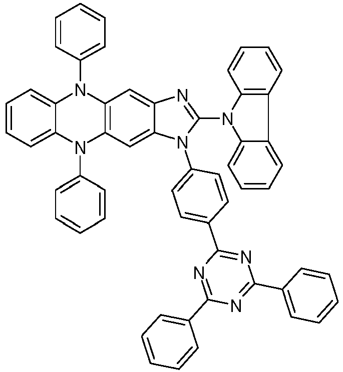 | 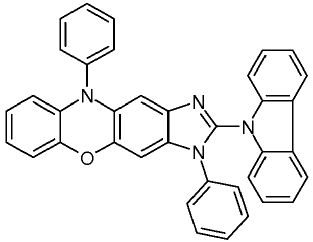 |
| 105 | 106 |
| 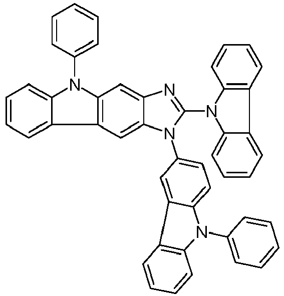 | 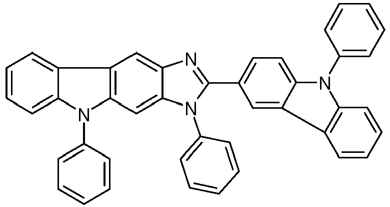 |
| 107 | 108 |
| 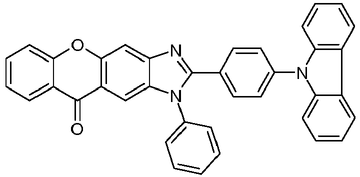 | 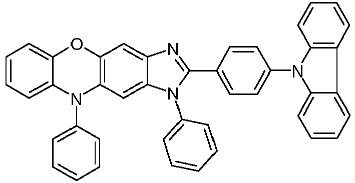 |
| 109 | 110 |
| 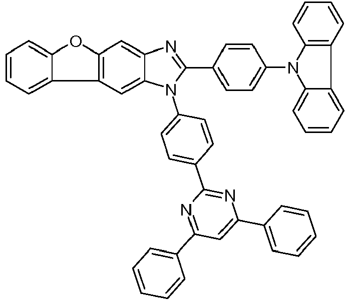 | 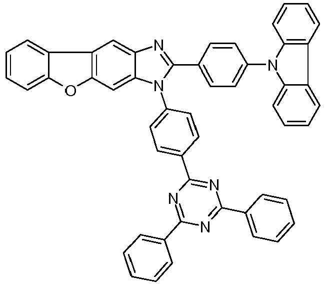 |
| 111 | 112 |
| 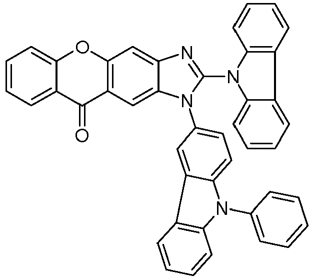 | 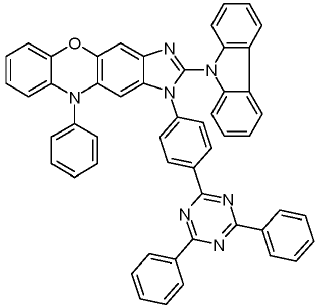 |
| 113 | 114 |
| 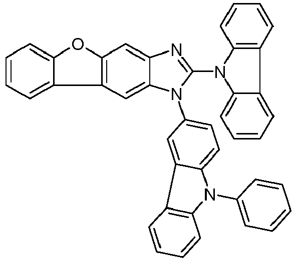 | 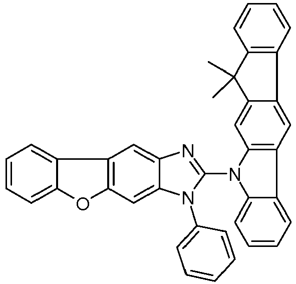 |
| 115 | 116 |
| 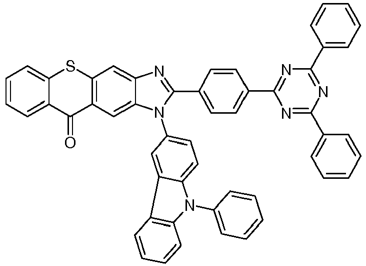 | 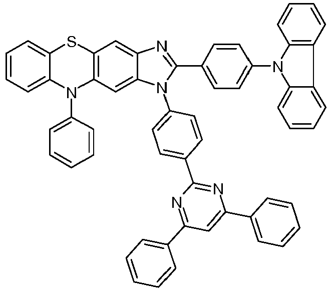 |
| 117 | 118 |
| 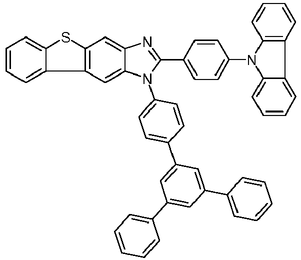 | 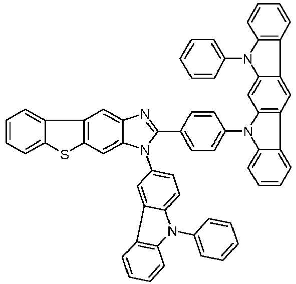 |
| 119 | 120 |
| 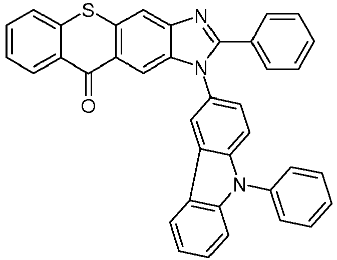 | 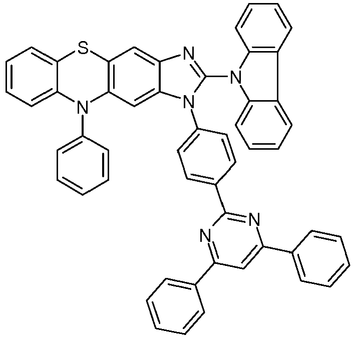 |
| 121 | 122 |
| 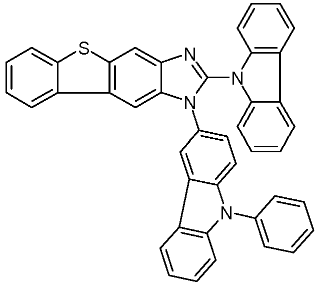 | 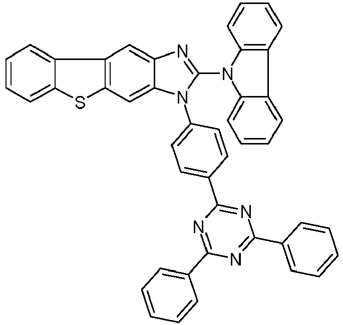 |
| 123 | 124 |

Die Verbindungen können gemäß im Stand der Technik bekannten Syntheseschritten hergestellt werden. Dabei kommen unter anderem metallkatalysierte Kupplungsreaktionen wie Buchwald-Kupplung und Suzuki-Kupplung zum Einsatz. Weiterhin kommen dabei Kondensationsreaktionen zur Bildung von heteroaromatischen Ringen zum Einsatz. Nochmals weiterhin kommen dabei nukleophile Substitutionsreaktionen an elektronenarmen Heteroaromaten zum Einsatz.

Ein möglicher, bevorzugter Weg zur Herstellung von Verbindungen gemäß Formel (I) ist im folgenden Schema 1 gezeigt.

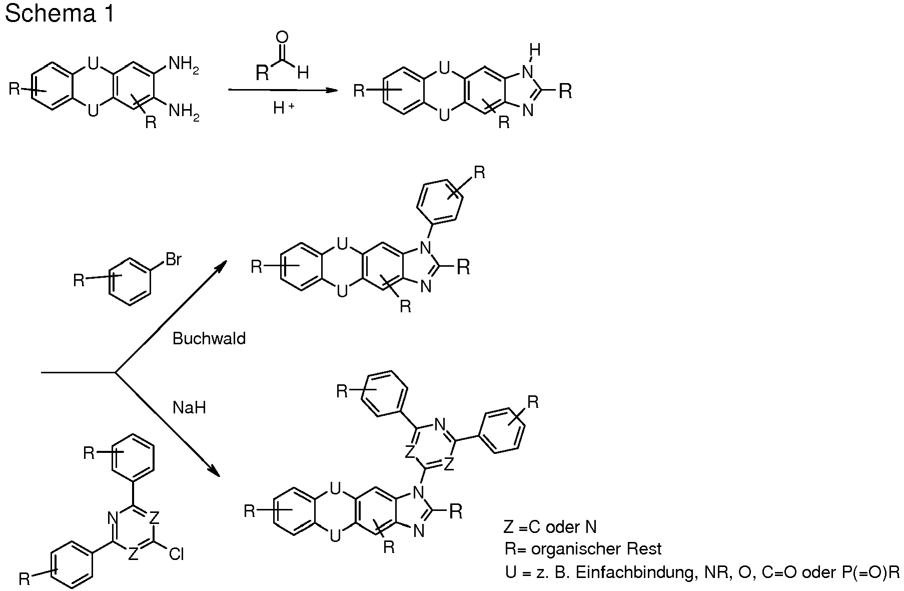

In einem ersten Schritt wird der heteroaromatische Fünfring über eine Kondensationsreaktion gebildet. Gezeigt ist die Synthese eines Imidazolrings, es können auf diese oder auf analoge Weise aber auch andere heteroaromatische Fünfringe in Verbindungen gemäß Formel (I) gebildet werden.

In einem folgenden Schritt kann entweder über eine Buchwald-Kupplung eine Aryl- oder Heteroarylgruppe in das Molekül eingeführt werden, oder es kann über eine nukleophile Substitutionsreaktion eine elektronenarme Heteroarylgruppe eingeführt werden, durch Deprotonierung am Stickstoffatom des Benzimidazols.

Auf diese Weise können am Grundkörper eine Vielzahl unterschiedlicher Aryl- oder Heteroarylgruppen eingeführt werden. Dabei können mehrere der oben genannten Kupplungsreaktionen durchgeführt werden, oder es kann eine einzige Kupplungsreaktion mehrfach durchgeführt werden, beispielsweise eine doppelte Buchwald-Kupplung bei Vorliegen von zwei Aminofunktionen.

Analog zu den in Schema 1 gezeigten Synthesen können die Verbindungen gemäß Formel (I) auch unter Variation der gezeigten Substituenten synthetisiert werden. Beispielsweise können anstelle von Triazin ähnliche Heterocyclen wie Pyridinyl und Pyrimidinyl eingeführt werden. Ebenso können anstelle des Phenylrests Biphenyl, Terphenyl, Naphthyl, Fluorenyl, Carbazolyl und ähnliche Substituenten eingeführt werden.

Weiterhin können sich an die Kupplungsreaktionen weitere Funktionalisierungsreaktionen anschließen, um zu der endgültigen Verbindung gemäß Formel (I) zu gelangen.

Eine weitere mögliche Methode zur Herstellung von Verbindungen gemäß Formel (I) ist im folgenden Schema 2 gezeigt.

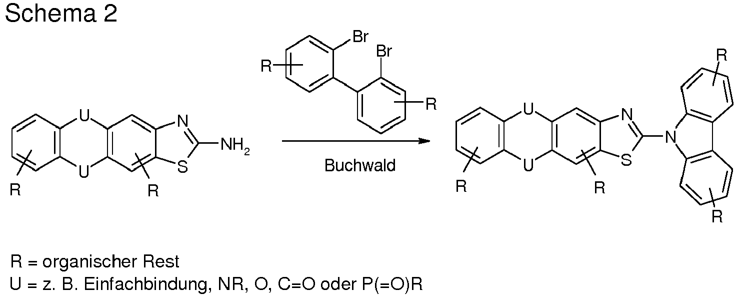

Dabei wird von einer heterocyclischen Verbindung ausgegangen, die eine primäre Aminogruppe trägt. Solche Verbindungen können entweder leicht hergestellt werden oder sind in vielen Fällen auch kommerziell erhältlich. Diese Verbindung wird anschließend in einer Buchwald-Reaktion mit einem Dibromid umgesetzt. Dadurch wird ein Carbazol-Derivat an den heteroaromatischen Grundkörper gebunden.

Somit ist ein Verfahren zur Herstellung einer Verbindung der Formel (I-1), dadurch gekennzeichnet, dass
zunächst der heteroaromatische Grundkörper hergestellt wird, bevorzugt unter Einsatz einer oder mehrerer Kondensationsreaktionen, und anschließend eine oder mehrere Aryl- oder Heteroarylgruppen eingeführt werden, bevorzugt unter Verwendung von Buchwald-Kupplungen oder nukleophilen aromatischen Substitutionsreaktionen.

Die oben beschriebenen Verbindungen nach Formel (I-1), insbesondere Verbindungen, welche mit reaktiven Abgangsgruppen, wie Brom, lod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, lod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterhin interessant sind Oligomere, Polymere oder Dendrimere enthaltend eine oder mehrere Verbindungen gemäß Formel (I-1), wobei die Bindung(en) zum Polymer, Oligomer oder Dendrimer an beliebigen, in Formel (I-1) mit R¹ oder R² substituierten Positionen lokalisiert sein können. Je nach Verknüpfung der Verbindung gemäß Formel (I-1) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I-1) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I-1) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I-1) in Oligomeren, Dendrimeren und Polymeren gelten dieselben Bevorzugungen wie oben für Verbindungen gemäß Formel (I-1) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I-1) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Weiterhin interessant ist eine Formulierung, insbesondere eine Lösung, Dispersion oder Emulsion, enthaltend mindestens eine Verbindung gemäß Formel (I-1) oder mindestens ein Polymer, Oligomer oder Dendrimer enthaltend mindestens eine Einheit gemäß Formel (I-1) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die Verbindungen gemäß Formel (I) eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt. Bevorzugt erfolgt die Verwendung der Verbindungen der Formel (I) in elektronentransportierenden Schichten und/oder in emittierenden Schichten. In letzteren erfolgt die Verwendung bevorzugt als Matrixmaterial, besonders bevorzugt als Matrixmaterial für phosphoreszierende Emitter.

Weiterer Gegenstand der Erfindung ist daher die Verwendung einer Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Gegenstand der Erfindung ist, wie bereits oben ausgeführt, eine elektronische Vorrichtung, enthaltend Anode, Kathode sowie mindestens eine organische Schicht enthaltend mindestens eine Verbindung gemäß Formel (I). Dabei ist die elektronische Vorrichtung bevorzugt ausgewählt aus den oben genannten Vorrichtungen. Besonders bevorzugt ist sie eine organische Elektrolumineszenzvorrichtung, enthaltend Anode, Kathode und mindestens eine emittierende Schicht, dadurch gekennzeichnet, dass mindestens eine organische Schicht, die eine emittierende Schicht, eine Elektronentransportschicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die Abfolge der Schichten der Vorrichtung ist bevorzugt die folgende: Anode-Lochinjektionsschicht-Lochtransportschicht-emittierende Schicht-Elektronentransportschicht-Elektroneninjektionsschicht-Kathode.

Dies gilt bevorzugt für Vorrichtungen, bei denen die Schichten durch Gasphasenabscheidung aufgetragen werden.

Für Vorrichtungen, bei denen die Schichten aus Lösung aufgetragen werden, wird bevorzugt die folgende Schichtabfolge verwendet: Anode-Lochinjektionsschicht-Interlayer-emittierende Schicht-Kathode. Dabei kann statt des Begriffs Lochinjektionsschicht auch synonym der Begriff Buffer-Layer verwendet werden. Dabei kann statt des Begriffs Interlayer auch synonym der Begriff Lochtransportsschicht verwendet werden. Es soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) enthält und wobei die drei Schichten blaue, grüne und orange oder rote Emission zeigen (für den prinzipiellen Aufbau siehe z. B. WO 2005/011013). Alternativ und/oder zusätzlich kann die erfindungsgemäße Verbindung auch in der Elektronentransportschicht oder in einer anderen Schicht vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Bevorzugt ist, dass die elektronische Vorrichtung die Verbindung der Formel (I) in einer Elektronentransportschicht enthält. Unter einer Elektronentransportschicht wird im Rahmen der vorliegenden Anmeldung eine Schicht verstanden, die zwischen Kathode und emittierender Schicht liegt.

In der Literatur sind als Untertypen von Elektronentransportschichten Lochblockierschichten und Elektroneninjektionsschichten bekannt. Diese werden im Sinne der vorliegenden Anmeldung als vom Begriff Elektronentransportschicht umfasst verstanden. Im Speziellen bezeichnet eine Lochblockierschicht eine direkt an die emittierende Schicht kathodenseitig angrenzende Schicht, die aus einem Material mit tiefliegendem HOMO besteht und die gute Elektronentransporteigenschaften aufweist. Das HOMO ist dabei bevorzugt tiefer liegend als das HOMO der emittierende Schicht. Unter einer Elektroneninjektionsschicht wird eine Schicht verstanden, die direkt an die Kathode angrenzt, und die den Eintritt von Elektronen aus der Kathode in das organische Material erleichtert.

Die Elektronentransportschicht enthaltend die Verbindung der Formel (I) kann die Verbindung als Reinmaterial oder in Kombination mit einer oder mehreren weiteren Verbindungen aufweisen. Wenn weitere Verbindungen vorhanden sind, so sind diese bevorzugt in geringem Anteil vorhanden. Solche Mindermengenkomponenten der Elektronentransportschicht werden als Dotanden bezeichnet, insbesondere als n-Dotanden, wenn sie starke Reduktionsmittel darstellen, bevorzugt wenn sie stärkere Reduktionsmittel darstellen als die Hauptkomponente der Elektronentransportschicht.

In einer weiteren Ausführungsform der vorliegenden Erfindung enthält die elektronische Vorrichtung die Verbindung der Formel (I) in einer emittierenden Schicht als Matrixmaterial in Kombination mit einem oder mehreren Dotanden, vorzugsweise phosphoreszierenden Dotanden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen. Beispiele für phosphoreszierende Dotanden sind in einem folgenden Abschnitt aufgeführt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung wird die Verbindung gemäß Formel (I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Bevorzugte phosphoreszierende Dotanden zur Verwendung in Mixed-Matrix-Systemen sind die im Folgenden angebenen bevorzugten phosphoreszierenden Dotanden.

Es folgen bevorzugte Ausführungsformen der Funktionsschichten und der Funktionsmaterialien der erfindungsgemäßen Vorrichtung:
Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Explizite Beispiele für phosphoreszierende Dotanden sind in der folgenden Tabelle aufgeführt.

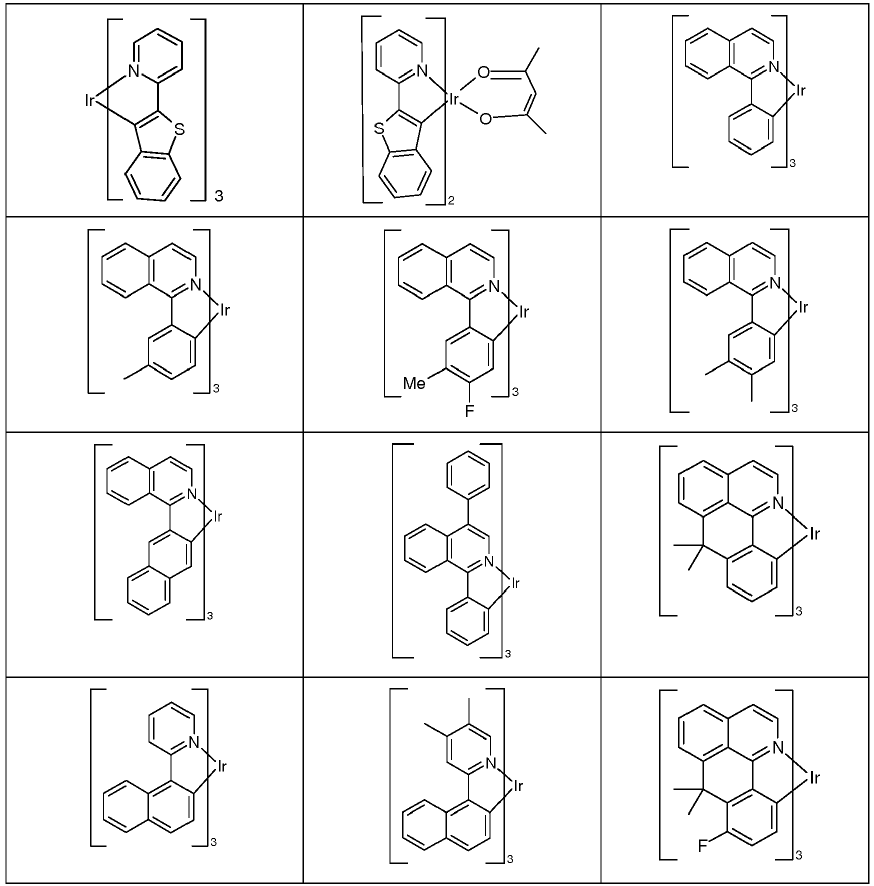

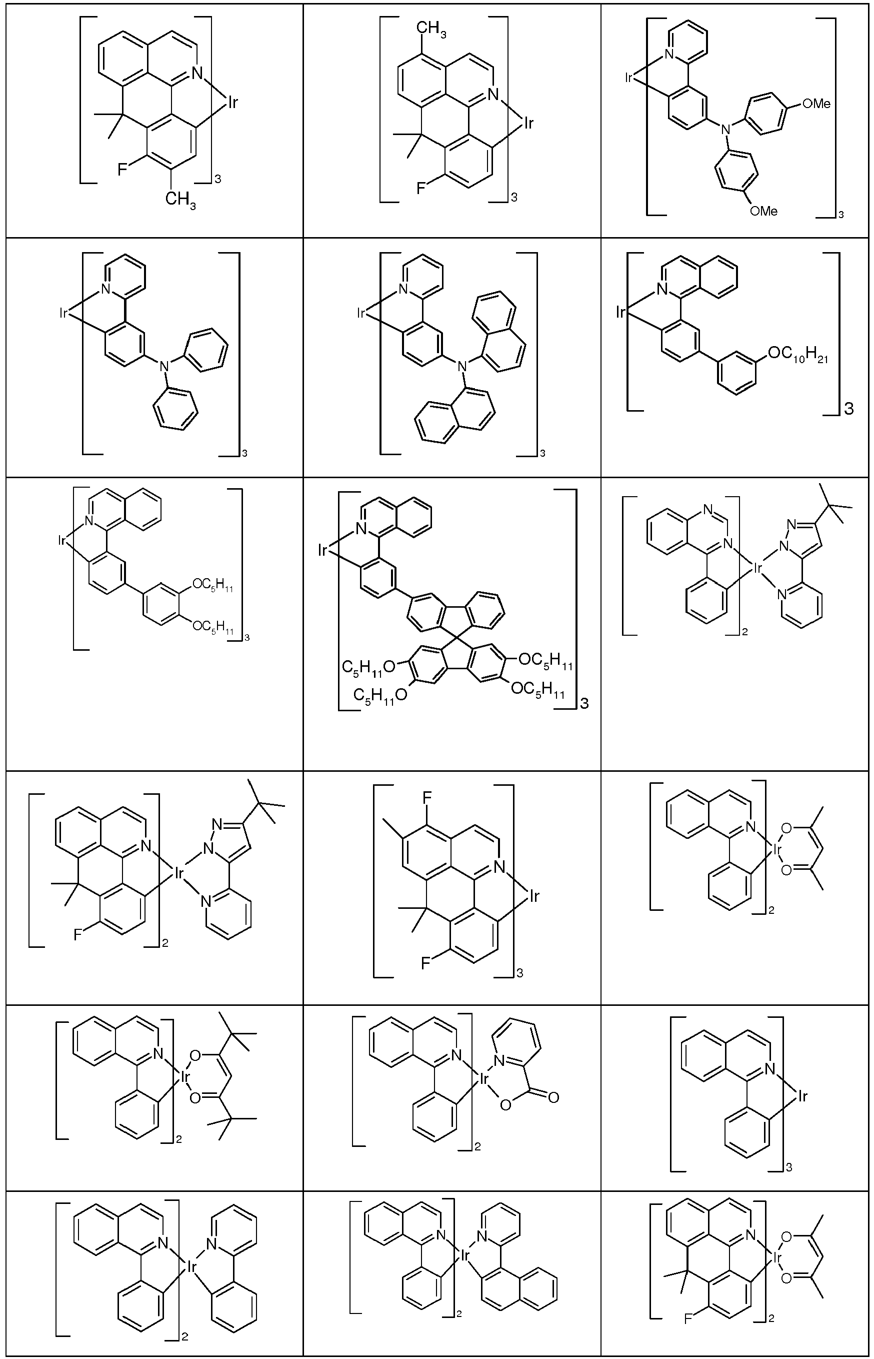

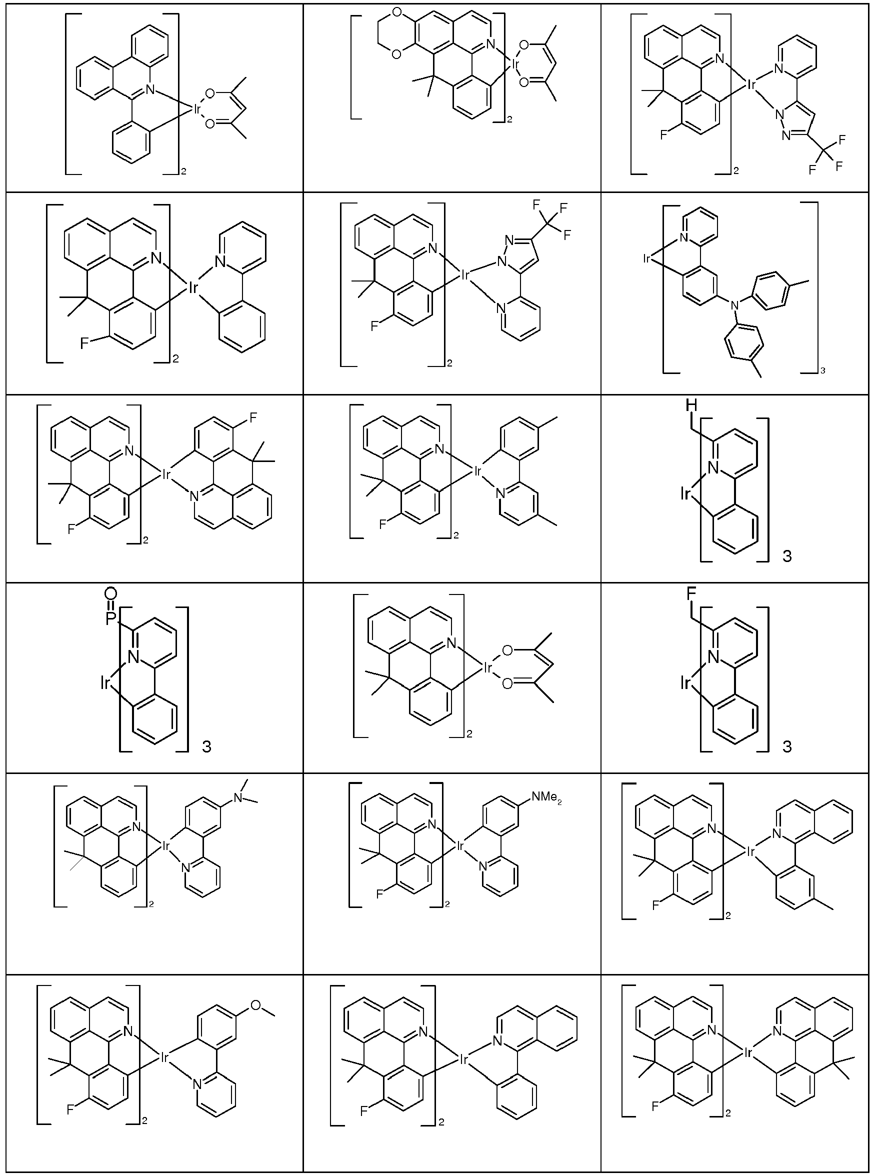

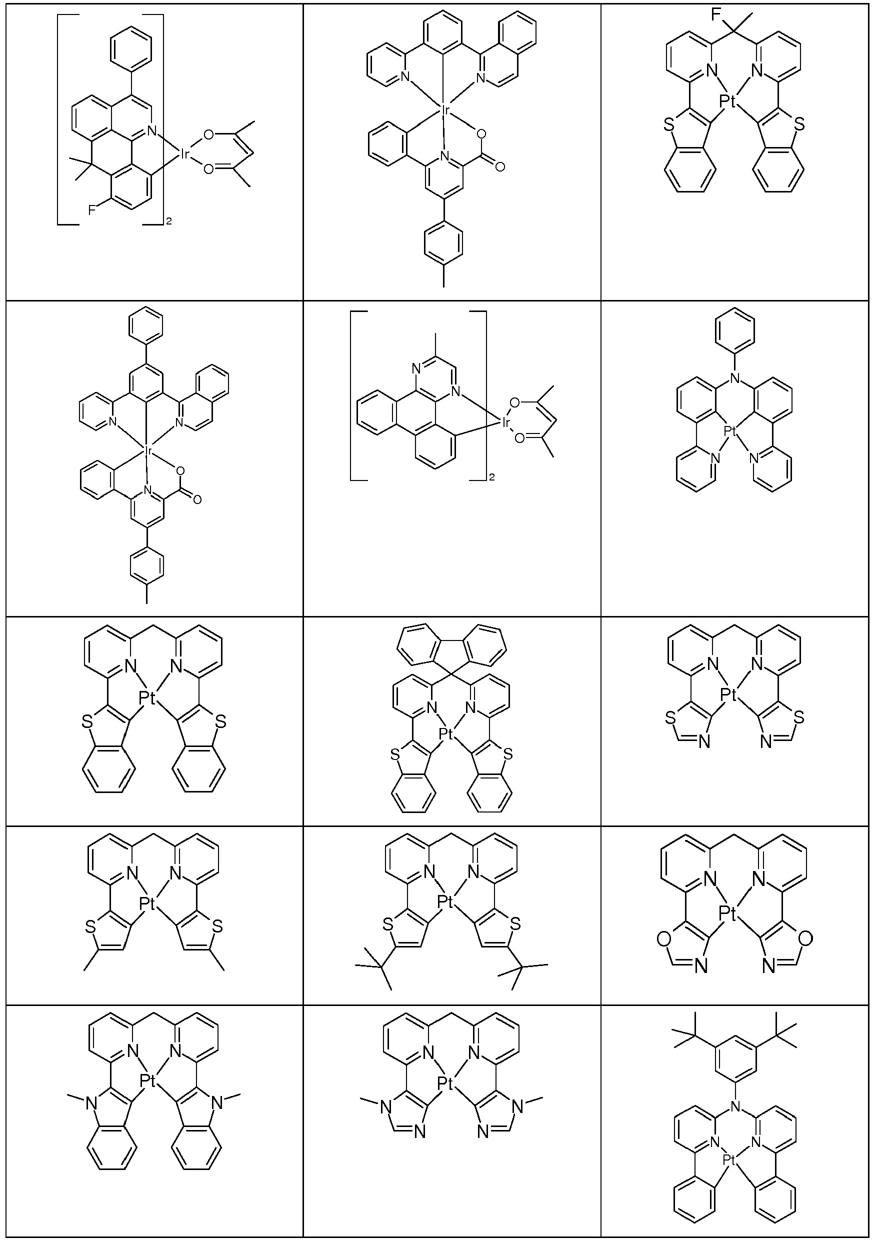

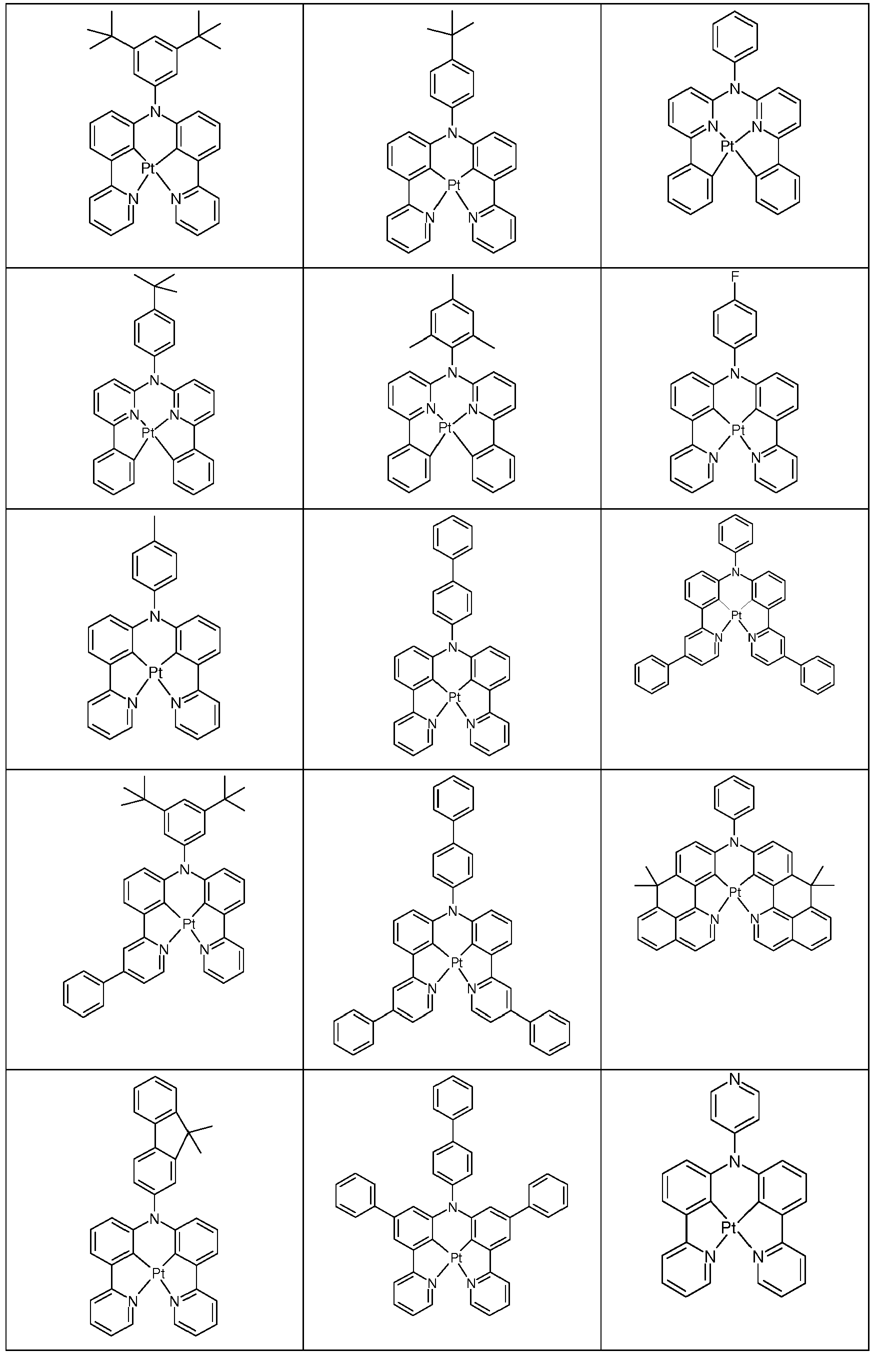

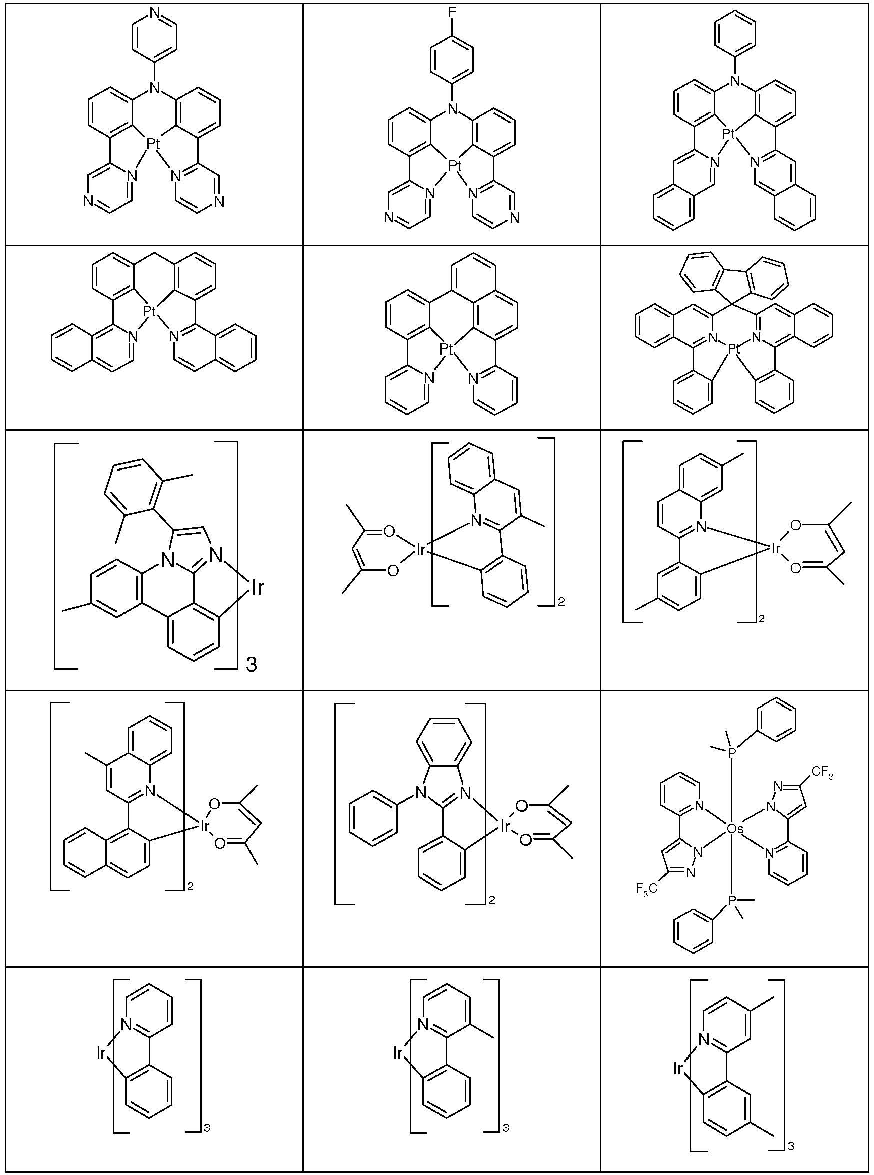

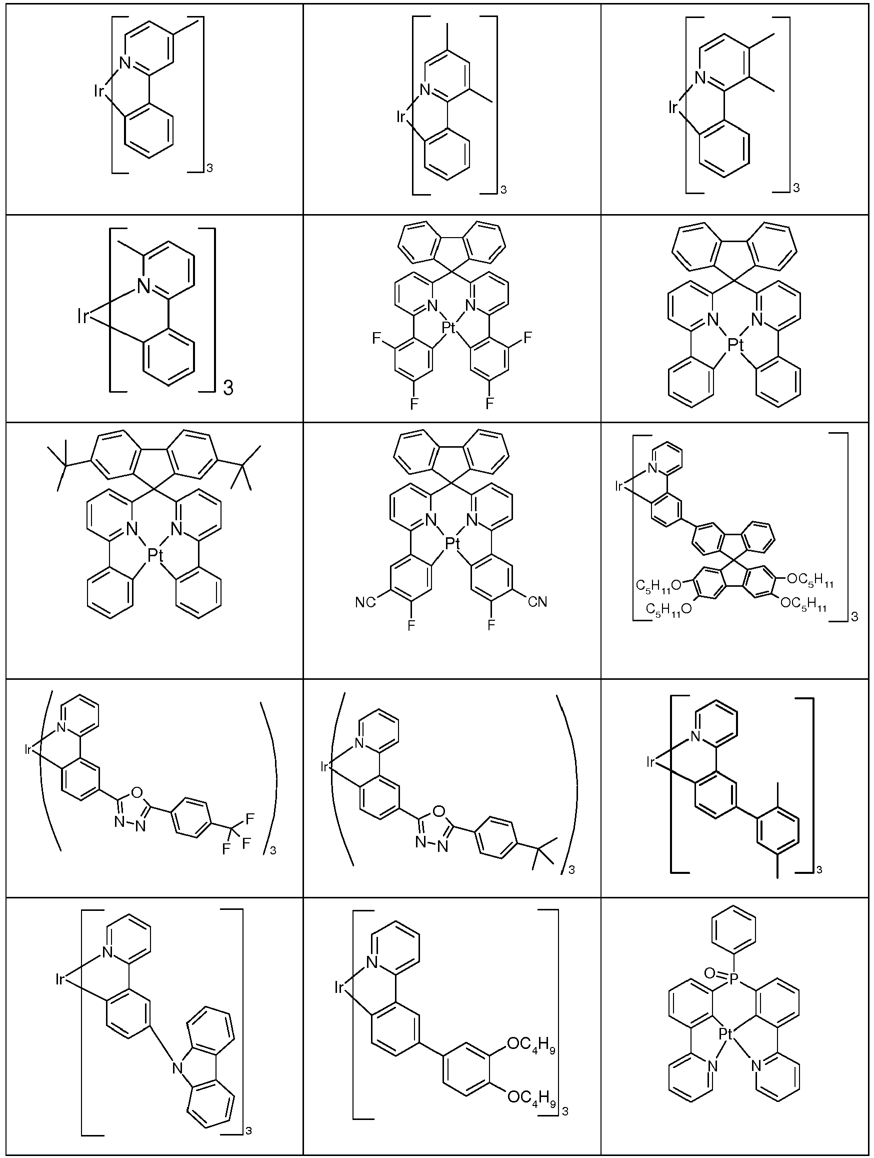

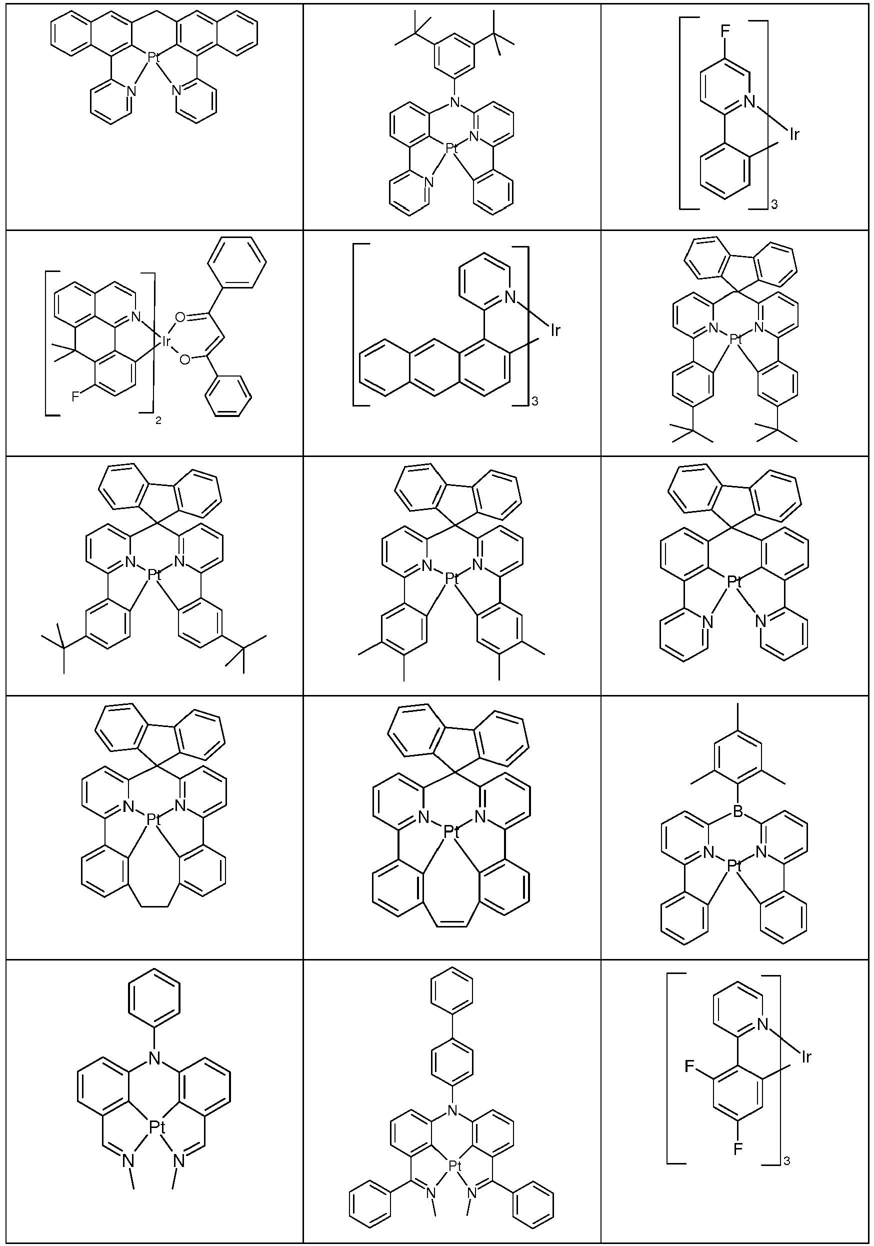

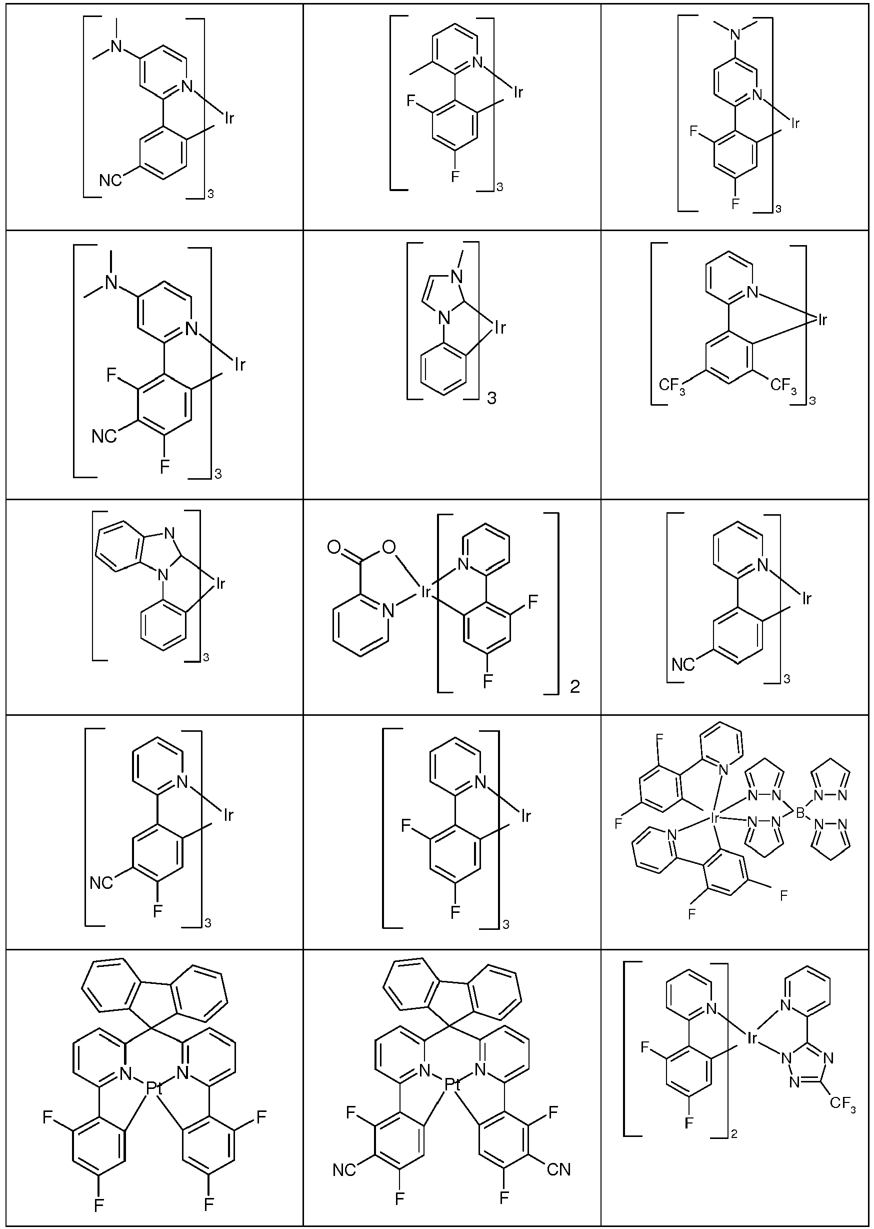

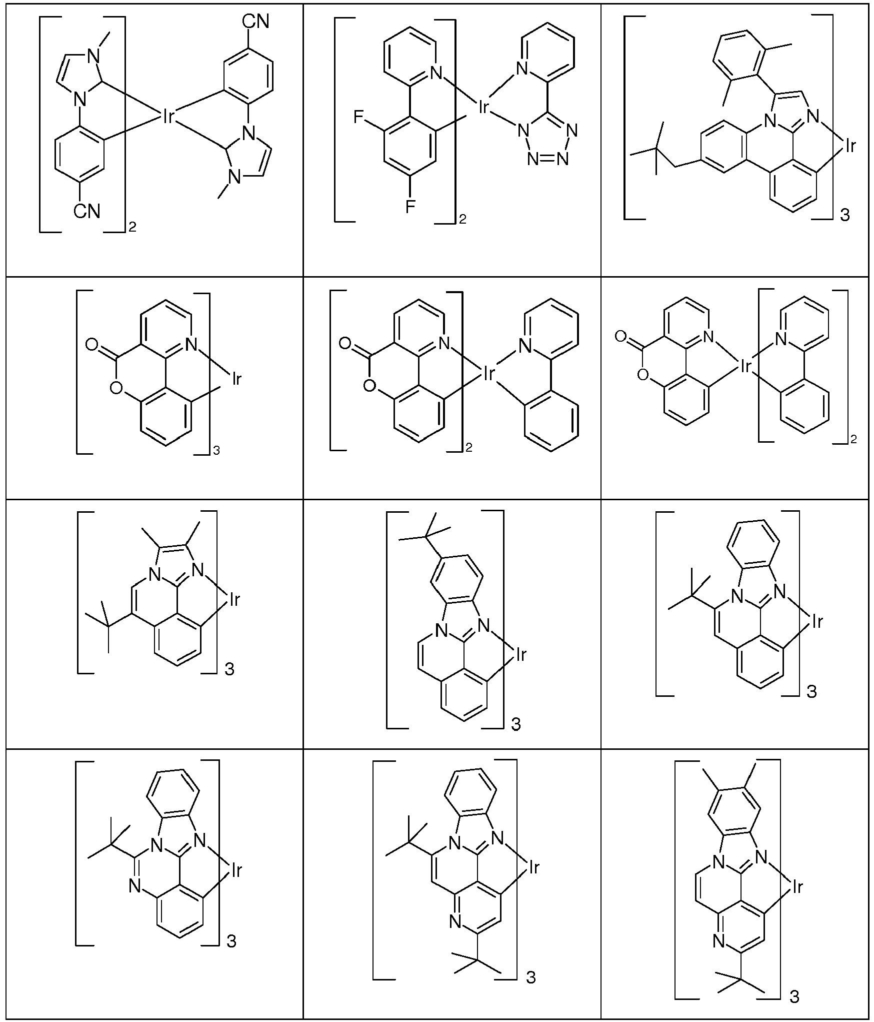

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen.

Als Matrixmaterialien, bevorzugt für fluoreszierende Dotanden, kommen neben den Verbindungen der Formel (I) Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den Verbindungen der Formel (I) aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729 und DiazaphospholDerivate, z. B. gemäß WO 2010/054730.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind neben den Verbindungen der Formel (I) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können neben den Verbindungen der Formel (I) alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Materialien, die in einer Lochtransport-, Lochinjektions- oder Elektronenblockierschicht der erfindungsgemäßen elektronischen Vorrichtung verwendet werden können, sind Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß WO 2013/083216) und Dihydroacridin-Derivate (z. B. gemäß WO 2012/150001).

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, AI, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die elektronische Vorrichtung wird bei der Herstellung entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

Ein weiterer Gegenstand der vorliegenden Erfindung bezieht sich daher auf die Verwendung der erfindungsgemäßen Verbindungen und Vorrichtungen enthaltend die Verbindungen zur Behandlung, Prophylaxe und Diagnose von Erkrankungen. Noch ein weiterer Gegenstand der vorliegenden Erfindung bezieht sich auf die Verwendung der erfindungsgemäßen Verbindungen und Vorrichtungen enthaltend die Verbindungen zur Behandlung und Prophylaxe von kosmetischen Umständen.

Details betreffend die Verwendung von organischen Elektrolumineszenzvorrichtungen in der Lichttherapie und in der Behandlung und Prophylaxe kosmetischer Umstände sind in WO 2011 /069590 und WO 2011/110277 offenbart. Die diesbezügliche Offenbarung wird in die Offenbarung der vorliegenden Anmeldung miteinbezogen.

Phototherapie oder Lichttherapie findet in vielen medizinischen und/oder kosmetischen Bereichen Anwendung. Die erfindungsgemäßen Verbindungen sowie die Vorrichtungen enthaltend diese Verbindungen können daher zur Therapie und/oder Prophylaxe und/oder Diagnose von allen Erkrankungen und/oder in kosmetischen Anwendungen eingesetzt werden, für die der Fachmann die Anwendung von Phototherapie in Betracht zieht. Der Begriff Phototherapie beinhaltet dabei neben der Bestrahlung auch die photodynamische Therapie (PDT) sowie das Desinfizieren und Sterilisieren im Allgemeinen.

### Ausführungsbeispiele

Die folgenden Ausführungsbeispiele dienen zur Erläuterung der Erfindung. Sie sollen nicht einschränkend ausgelegt werden.

### A) Synthesebeispiele

Die nachfolgenden Synthesen werden, sofern nicht anders angegeben, unter einer Schutzgasatmosphäre in getrockneten Lösungsmitteln durchgeführt.

### Synthese von Vorstufen:

### Beispiel Int-1a: 2-Phenyl-3,9-dihydro-1,3,9-triaza-cyclopenta[b]fluoren

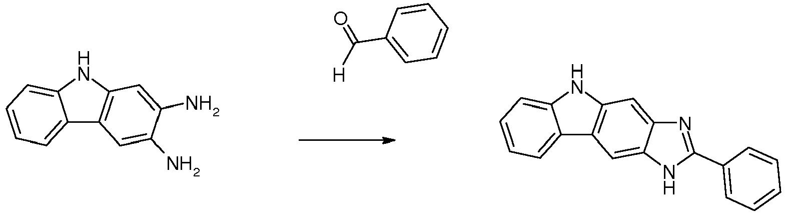

5 g (50 mmol) Benzaldehyd wird tropfenweise zu 9,8 g (50 mmol) 9H-Carbazol-2,3-diamin in 300 ml DMF und 10 ml konz. Schwefelsäure gegeben und für 2 h bei Raumtemperatur gerührt. Die Mischung wird auf 500 g Eis gegeben und mit Dichloromethan extrahiert. Die organische Phase wird mit 4 x 50 mL H₂O gewaschen, über MgSO₄ getrocknet und die Lösungsmittel im Vakuum entfernt. Das reine Produkt erhält man durch Umkristallisation. Der Gehalt an Produkt beträgt gemäß HPLC 98 % bei einer Gesamtausbeute von 7,3 g (25 mmol, 52 %).

Analog können folgende Verbindungen erhalten werden:

| **Bsp.** | **Edukt** | **Edukt** | **Produkt** |
|---|---|---|---|
| Int-1b | 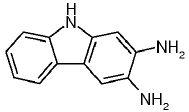 | 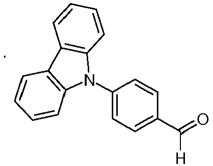 | 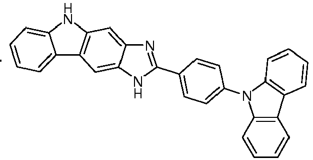 |
| | 86439-50-1 | 110677-45-7 | |
| Int-1c | 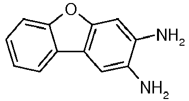 | 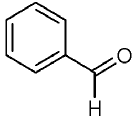 | 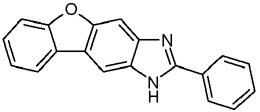 |
| | 24258-73-9 | | |
| Int-1d | 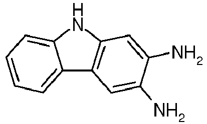 | 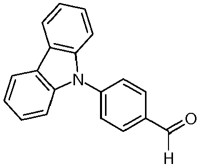 | 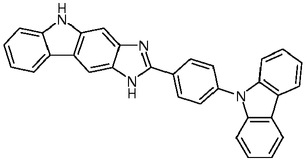 |
| | 86439-50-1 | | |
| Int-1e | 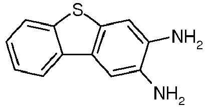 | 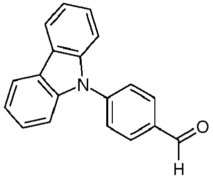 | 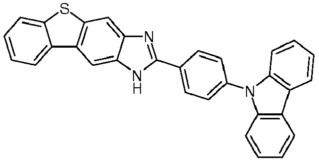 |
| | 1 06020-19-3 | | |

| **Bsp.** | **Ausbeute** |
|---|---|
| Int-1b | 54% |
| Int-1c | 49 % |
| lnt-1d | 66% |
| Int-1e | 53% |

### Synthese von erfindungsgemäßen Verbindungen:

### Beispiel 2a: 2-(4-Carbazol-9-yl-phenyl)-3-(4,6-diphenyl-pyrimidin-2-yl)-3H-9-thia-1,3-diaza-cyclopenta[b]fluoren

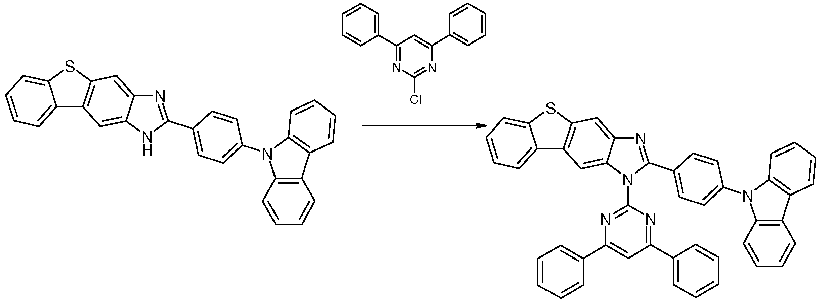

13,1g (28,2 mmol) 2-(4-Carbazol-9-yl-phenyl)-3H-9-thia-1,3-diazacyclopenta[b]fluoren werden in 225 ml Dimethylformamid unter Schutzgasatmosphäre gelöst und mit 1,5 g NaH, 60%ig in Mineralöl, (37,5 mmol) versetzt. Nach 1 h bei Raumtemperatur wird eine Lösung von 2-Chlor-4,6-diphenyl-[1,3,5]-triazin (8,5 g, 31,75 mmol) in 75 mL Dimethylformamid zugetropft. Das Reaktionsgemisch wird dann 12 h bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert und aus Toluol umkristallisiert, abschließend im Hochvakuum sublimiert. Die Reinheit beträgt 99,9%. Die Ausbeute beträgt 14,8 g (21 mmol, 80%).

Analog können folgende Verbindungen erhalten werden:

| **Bsp.** | **Edukt** | **Edukt** | **Produkt** |
|---|---|---|---|
| 2b | 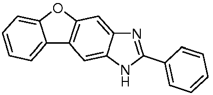 | 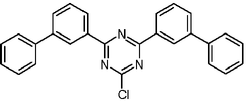 | 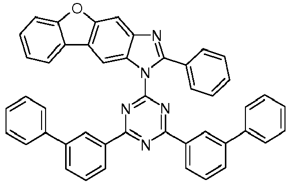 |
| 2c | 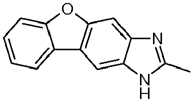 | 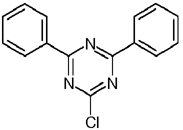 | 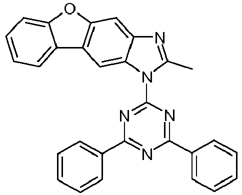 |
| | 857550-84-6 | | |

| **Bsp.** | **Ausbeute** |
|---|---|
| 2b | 58% |
| 2c | 67% |

Analog können folgende Verbindungen mit 2 eq. NaH und 2eq. 2-Chlor-4,6-diphenyl-[1,3,5]-triazin erhalten werden:

| **Bsp.** | **Edukt** | **Edukt** | **Produkt** |
|---|---|---|---|
| 2d | 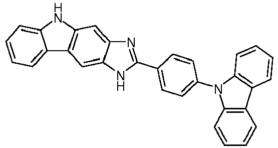 | 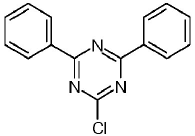 | 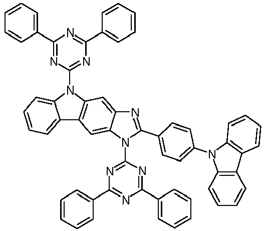 |
| 2e | 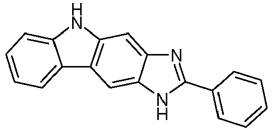 | 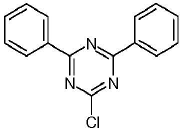 | 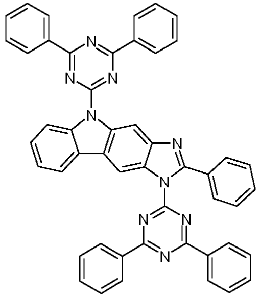 |
| 2f | 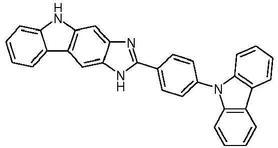 | 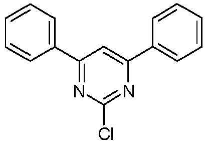 | 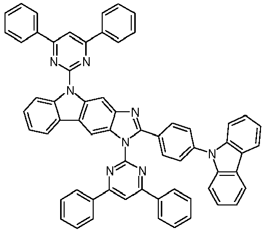 |
| | | 2 eq. | |

| **Bsp.** | **Ausbeute** |
|---|---|
| 2d | 69% |
| 2e | 77% |
| 2f | 73% |

### Beispiel 3a: 2-Phenyl-1-(9-phenyl-9H-carbazol-3-yl)-1H-benzo[4',5']furo[2',3':4,5]benzo [1,2-d]imidazol

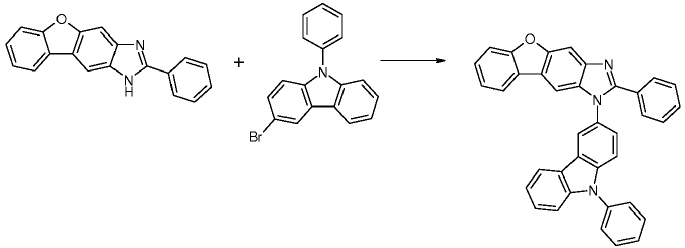

13,5 g (42,12 mmol) 3-Bromo-9-phenyl-9H-carbazol, 13,2 g (47 mmol) 2-Phenyl-1H-benzo[4',5']furo[2',3':4,5]benzo[1,2-d]imidazol und 29,2 g Rb₂CO₃ werden in 250 mL p-Xylol suspendiert. Zu dieser Suspension werden 0,95 g (4,2 mmol) Pd(OAc)₂ und 12,6 ml einer 1M Tri-tert-butylphosphinlösung gegeben. Die Reaktionsmischung wird 16 h unter Rückfluss erhitzt. Nach Erkalten wird die organische Phase abgetrennt, dreimal mit 200 mL Wasser gewaschen und anschließend zur Trockene eingeengt. Der Rückstand wird mit Toluol heiß extrahiert, aus Toluol umkristallisiert und abschließend im Hochvakuum sublimiert. Die Reinheit beträgt 99,9%. Ausbeute: 18 g (34 mmol), 76 % der Theorie.

Analog können folgende Verbindungen erhalten werden:

| **Bsp.** | **Edukt** | **Edukt** | **Produkt** |
|---|---|---|---|
| 3b | 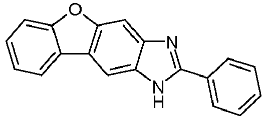 | 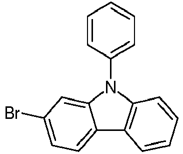 94994-62-4 | 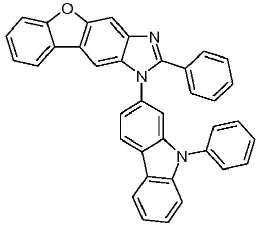 |
| 3c | 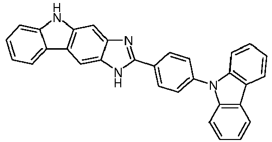 | 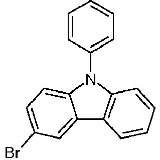 | 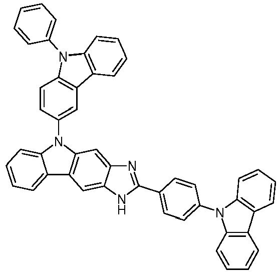 |
| 3d | 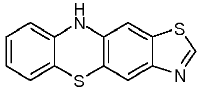 21426-61-9 | 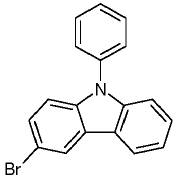 | 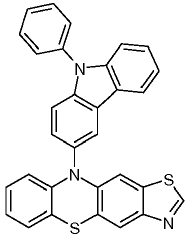 |
| 3e | 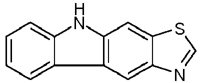 242-93-3 | 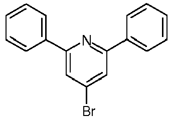 78500-89-7 | 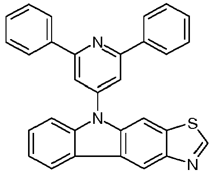 |
| 3f | 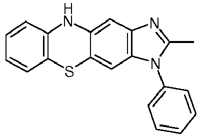 823802-18-2 | 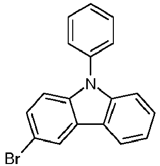 | 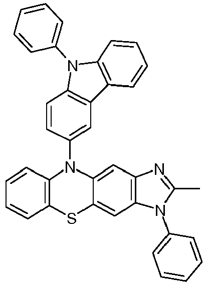 |
| 3g | 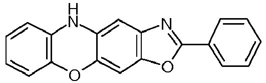 59225-35-3 | 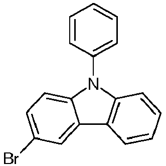 | 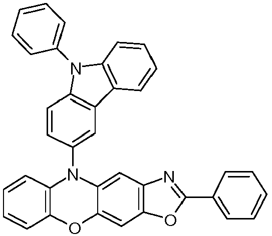 |

| **Bsp.** | **Ausbeute** |
|---|---|
| 3b | 76% |
| 3c | 85% |
| 3d | 56% |
| 3e | 76% |
| 3f | 41% |
| 3g | 53% |

Analog können folgende Verbindungen mit 2 eq. 3-Bromo-9-phenyl-9H-carbazol erhalten werden:

| **Bsp.** | **Edukt** | **Edukt** | **Produkt** |
|---|---|---|---|
| 3h | 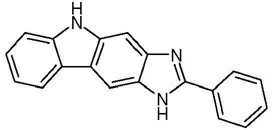 | 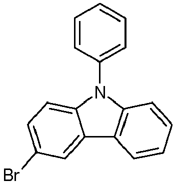 | 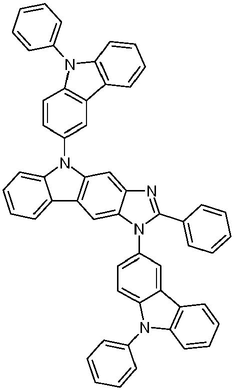 |
| 3j | 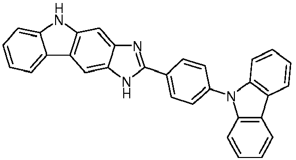 | 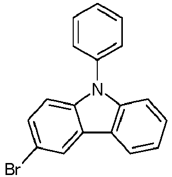 | 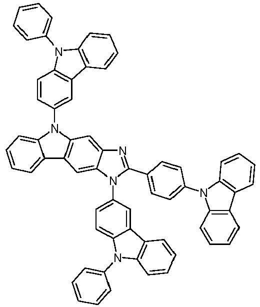 |

| **Bsp.** | **Ausbeute** |
|---|---|
| 3h | 64% |
| 3j | 55% |

### Beispiel 4a: 2-Carbazol-9-yl-9-oxa-3-thia-1-aza-cyclopenta[b]fluoren

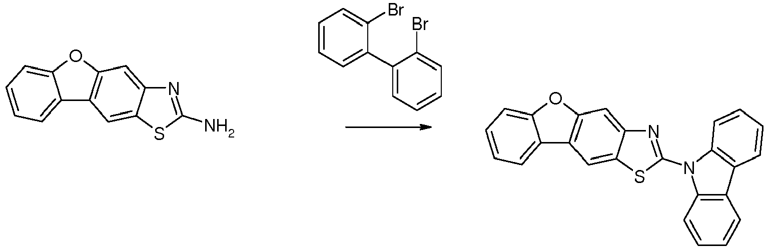

15,6 g (50 mmol) 2,2 Dibrom-1,1 -biphenyl werden mit 500 ml Toluol, 2,3 g (2,5 mmol) Tris(dibenzylidenaceton)dipalladium(0), 10 mL 1M t-Bu₃P in Toluol und Natrium-tert-butoxid 11,5 g (120 mmol) versetzt. Anschließend werden 9,6 g (40 mmol) 9-Oxa-3-thia-1-aza-cyclopenta[b]fluoren-2-ylamin zugegeben. Der Ansatz wird 20 h auf 110 °C erhitzt, dann auf Raumtemperatur abgekühlt und 400 ml Wasser zugegeben. Es wird mit Essigester extrahiert, danach werden die vereinigten organischen Phasen über Natriumsulfat getrocknet, und unter vermindertem Druck eingeengt. Der Rückstand wird aus Toluol und aus Dichlormethan/iso-Propanol umkristallisiert. Die Ausbeute beträgt 8,5 g (22 mmol), entsprechend 55 % der Theorie.

Analog können folgende Verbindungen erhalten werden:

| **Bsp.** | **Edukt** | **Edukt** | **Produkt** | **Ausbeute** |
|---|---|---|---|---|
| 4b | 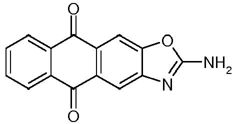 97339-25-8 | 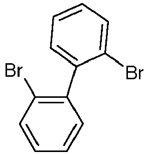 | 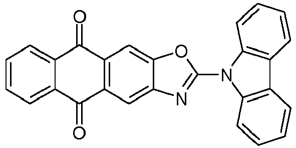 | 61% |
| 4c | 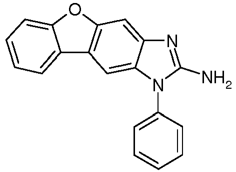 | 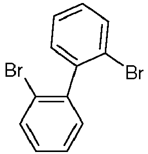 | 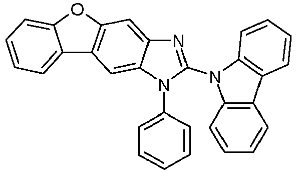 | 58% |

Andere Referenz-Materialien sind:

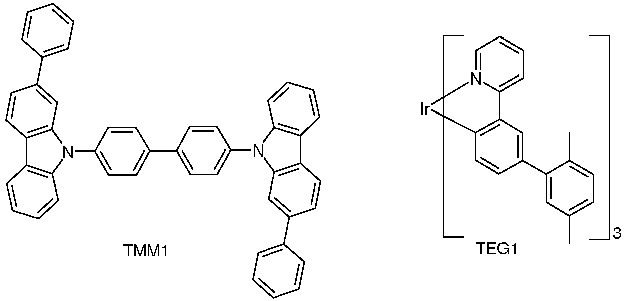

### B) Quantenchemische Simulationen zu erfindungsgemäßen Verbindungen und Referenz-Materialien

Die HOMO- und LUMO-Lagen sowie das Triplett/Singlett Niveau der organischen Verbindungen werden über quanten-chemische Rechnungen bestimmt. Hierzu wird das Programmpaket "Gaussian03W" (Gaussian Inc.) verwendet. Zur Berechnung organischer Substanzen ohne Metalle wird zuerst eine Geometrieoptimierung mit einer Semi-empirischen Methode "Ground State/Semi-empirical/ Default Spin/AM1" (Charge O/Spin Singlet) durchgeführt. Im Anschluss erfolgt auf Grundlage der optimierten Geometrie eine Energierechnung. Hierbei wird die Methode "TD-SCF/DFT/ Default Spin/B3PW91" mit dem Basissatz "6-31G(d)" verwendet (Charge 0/Spin Singlet). Die wichtigsten Ergebnisse sind HOMO / LUMO-Niveaus und Energien für die Triplett-und Singulett-angeregten Zustände. Der erste angeregte Singulett- und der erste angeregte Triplett-Zustand sind am wichtigsten und werden als T1 und S1 benannt. Aus der Energierechnung erhält man das HOMO HEh bzw. LUMO LEh in Hartree-Einheiten. Daraus werden die HOMO- und LUMO-Werte in Elektronenvolt wie folgt bestimmt, wobei sich diese Beziehungen aus der Kalibrierung anhand von Cyclovoltammetriemessungen ergeben:
HOMO(eV) = ((HEh^{∗}27.212)-0.9899)/1.1206
LUMO(eV) = ((LEh^{∗}27.212)-2.0041)/1.385

Diese Werte sind im Sinne dieser Anmeldung als energetische Lage des HOMO-Niveaus bzw. des LUMO-Niveaus der Materialien anzusehen. Als Beispiel erhält man für die Verbindung 2a (siehe auch Tabelle 1) aus der Rechnung ein HOMO von -0.20047 Hartrees und ein LUMO von -0.07772 Hartrees, was einem kalibrierten HOMO von -5.75 eV und einem kalibrierten LUMO von -2.97 eV entspricht.

Die folgenden Beispiele zeigen, wie durch quantenchemische Rechnungen aus Verbindungen mit der gemeinsamen erfindungsgemäßen Grundstruktur für die verschiedenen Verwendungen in einer OLED (als ETM, als HTM, als TMM) jeweils optimal geeignete Verbindungen identifiziert werden können.

**Tabelle 1: Zusammenfassung der Energieniveaus der erfindungsgemäßen Verbindungen und TMM1 als Referenz**

| | HOMO [eV] | LUMO [eV] | T1 [eV] | bevorzugte Verwendungen |
|---|---|---|---|---|
| TMM1 | -5,68 | -2,39 | 2,84 | Matrix & ETM |
| 2a | -5,75 | -2,97 | 2,52 | ETM |
| 2b | -5,99 | -2,95 | 2,59 | Matrix & ETM |
| 2c | -6,02 | -2,98 | 2,66 | Matrix & ETM |
| 2d | -5,80 | -2,94 | 2,48 | ETM |
| 2e | -5,95 | -2,90 | 2,54 | ETM |
| 2f | -5,65 | -2,79 | 2,55 | ETM |
| 3a | -5,82 | -2,42 | 2,70 | Matrix |
| 3b | -5,87 | -2,49 | 2,70 | Matrix |
| 3c | -5,42 | -2,54 | 2,52 | HTM |
| 3d | -5,15 | -2,34 | 2,56 | Matrix & HTM |
| 3e | -5,97 | -2,48 | 2,86 | Matrix |
| 3f | -4,90 | -2,20 | 2,56 | Matrix & HTM |
| 3g | -5,20 | -2,55 | 2,26 | HTM |
| 3h | -5,30 | -2,30 | 2,67 | Matrix |
| 3j | -5,33 | -2,40 | 2,59 | Matrix &HTM |
| 4a | -5,96 | -2,60 | 2,70 | Matrix |
| 4b | -6,33 | -3,56 | 2,34 | ETM |
| 4c | -5,76 | -2,60 | 2,63 | Matrix |

Das T1 -Niveau von TEG1 ist 2,52eV, welches vom Onset des Photolumineszenzspektrums von TEG1 in Toluol abgeleitet wurde. Ein optimales Matrix-Material für TEG1 sollte daher ein T1 -Niveau von > 2,52eV, vorzugsweise ≥ 2,57eV aufweisen. Daher sind besonders geeignet für diese Verwendung diejenigen erfindungsgemäßen Verbindungen aus Tabelle 1, bei denen unter "bevorzugter Verwendung" Matrix aufgeführt ist.

Für Verwendung als ETM sollte die Verbindung optimalerweise ein LUMO < -2,6 eV haben, daher sind besonders geeignet diejenigen erfindungsgemäßen Verbindungen aus Tabelle 1, bei denen unter "bevorzugter Verwendung" ETM aufgeführt ist. Und als HTM sollte die Verbindung bevorzugt ein HOMO ≥ -5,45 eV haben, daher sind besonders geeignet diejenigen erfindungsgemäßen Verbindungen aus Tabelle 1, bei denen unter "bevorzugter Verwendung" HTM aufgeführt ist.

### C) Device-Beispiele

### Herstellung von Lösungen und Zusammensetzungen enthaltend Matrixmaterialien und TEG1 zur Verwendung in OLEDs

Lösungen, wie sie in Tabelle 2 zusammengefasst sind, werden wie folgt hergestellt: Zunächst werden 200 mg des Matrixmaterials und 50 mg TEG1 in 10 mL Chlorbenzol gelöst und so lange gerührt, bis die Lösung klar ist. Die Lösung wird filtriert unter Verwendung eines Filters Millipore Millex LS, Hydrophobic PTFE 5.0 µm.

**Tabelle 2: Zusammensetzung der Lösungen**

| | Zusammensetzung | Verhältnis (bezogen auf Gewicht) | Konzentration |
|---|---|---|---|
| Lösung-Ref | TMM1 + TEG1 | 75% : 25% | 25 mg/ml |
| Lösung 1 | 2b + TEG1 | 75% : 25% | 25 mg/ml |
| Lösung 2 | 2c + TEG1 | 75% : 25% | 25 mg/ml |
| Lösung 3 | 3a + TEG1 | 75% : 25% | 25 mg/ml |
| Lösung 4 | 3b + TEG1 | 75% : 25% | 25 mg/ml |
| Lösung 5 | 3d + TEG1 | 75% : 25% | 25 mg/ml |
| Lösung 6 | 3e + TEG1 | 75% : 25% | 25 mg/ml |
| Lösung 7 | 3f + TEG1 | 75% : 25% | 25 mg/ml |
| Lösung 8 | 3h + TEG1 | 75% : 25% | 25 mg/ml |
| Lösung 9 | 3j + TEG1 | 75% : 25% | 25 mg/ml |
| Lösung 11 | 4a + TEG1 | 75% : 25% | 25 mg/ml |
| Lösung 12 | 4c + TEG1 | 75% : 25% | 25 mg/ml |

Die Lösungen werden verwendet, um die emittierende Schicht von OLEDs herzustellen. Die entsprechende Feststoffzusammensetzung kann erhalten werden, indem das Lösemittel der Lösungen verdampft wird. Dieses kann für die Herstellung weiterer Formulierungen verwendet werden.

### Herstellung der OLEDs

### OLED-Ref bis OLED12 mit der Struktur

ITO/PEDOT/Interlayer/EML/Kathode, werden unter Verwendung der entsprechenden Lösungen, wie in Tabelle 2 zusammengefasst, gemäß der folgenden Vorschrift hergestellt:
1) Beschichtung von 80 nm PEDOT (Clevios™ P VP Al 4083) auf ein ITObeschichtetes Glassubstrat durch Spin-Coating, und 10 Minuten bei 120°C Ausheizen.
2) Beschichtung einer 20 nm Interlayer durch Spin-Coating einer Toluollösung von HIL-012 (Merck KGaA) (Konzentration 0.5 Gew.%) in einer Glovebox.
3) Ausheizen der Interlayer bei 180 °C für 1 h in einer Glovebox.
4) Beschichtung einer 80 nm emittierenden Schicht durch Spin-Coating einer entsprechenden Lösung gemäß Tabelle 2.
5) Ausheizen der Vorrichtung bei 180 °C für 10 min.
6) Aufdampfen einer Ba/Al-Kathode (3 nm + 150 nm).
7) Verkapselung der Vorrichtung.

### Messungen und Vergleich der Ergebnisse

Die so erhaltenen OLEDs werden nach Standardmethoden charakterisiert. Dabei werden die folgenden Eigenschaften gemessen: UIL-Charakteristik, Elektrolumineszenzspektrum, Farbkoordinaten, Effizienz und Betriebsspannung. Die Ergebnisse sind in Tabelle 3 zusammengefasst, wobei OLED-Ref als Vergleich gemäß dem Stand der Technik dient. In Tabelle 3 steht Uₒₙ für die Einsatzspannung, und U(100) für die Spannung bei 100 cd/m².

**Tabelle 3: Messergebnisse der erfindungsgemäßen OLEDs und der Vergleichsbeispiele**

| | Max. Eff. [cd/A] | Uₒₙ [V] | U(100) [V] | ClEx @ 100 cd/m² | ClEy @ 100 cd/m² |
|---|---|---|---|---|---|
| OLED-Ref | 8,2 | 3,8 | 6,5 | 0,33 | 0,62 |
| OLED1 | 26,1 | 3,1 | 4,9 | 0,34 | 0,62 |
| OLED2 | 24,5 | 2,5 | 3,6 | 0,33 | 0,62 |
| OLED3 | 16,4 | 3,0 | 4,9 | 0,34 | 0,63 |
| OLED4 | 14,6 | 3,1 | 5,2 | 0,34 | 0,62 |
| OLED5 | 13,4 | 3,1 | 4,9 | 0,33 | 0,62 |
| OLED6 | 14,3 | 3,3 | 5,4 | 0,34 | 0,62 |
| OLED7 | 10,4 | 2,8 | 4,4 | 0,30 | 0,58 |
| OLED8 | 12,9 | 2,5 | 3,6 | 0,33 | 0,62 |
| OLED9 | 14,7 | 3,3 | 5,4 | 0,34 | 0,62 |
| OLED11 | 23,0 | 2,8 | 4.3 | 0,34 | 0,62 |
| OLED12 | 27,0 | 2,7 | 3,8 | 0,33 | 0,62 |

Wie aus Tabelle 3 ersichtlich ist, werden unter Verwendung der erfindungsgemäßen Verbindungen als Matrixmaterialien durchweg deutlich verbesserte phosphoreszierende OLEDs in Bezug auf Betriebsspannung und Effizienz erhalten. Alle OLEDs zeigen vergleichbare Farbkoordinaten.

Die beobachteten technischen Effekte sind nicht auf das gezeigte System beschränkt. Sie können beispielsweise auch mit anderen phosphoreszierenden Emittern und bei Verwendung von zusätzlichen Co-Matrices erzielt werden.

## Patentansprüche

1. Elektronische Vorrichtung, enthaltend Anode, Kathode und mindestens eine organische Schicht enthaltend mindestens eine Verbindung der Formel (I) 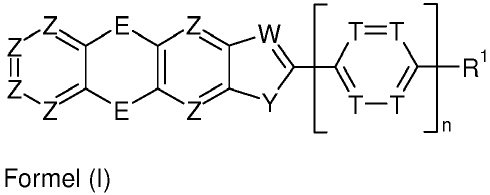 wobei gilt:
E ist bei jedem Auftreten gleich oder verschieden gewählt aus einer Einfachbindung, B(R¹), C=O, N(R¹), P(R¹), P(=O)R¹, O, S, S=O und S(=O)₂, wobei nicht beide Gruppen E eine Einfachbindung sein dürfen;
T ist bei jedem Auftreten gleich oder verschieden CR¹ oder N;
W ist CR¹ oder N;
Y ist N(R¹), O oder S;
Z ist bei jedem Auftreten gleich oder verschieden CR² oder N;
R¹ ist bei jedem Auftreten gleich oder verschieden H, D, F, C(=O)R³, CN, Si(R³)₃, N(R³)₂, P(=O)(R³)², S(=O)R³, S(=O)₂R³, eine geradkettige Alkyl-, oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch-R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F oder CN ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann;
R² ist bei jedem Auftreten gleich oder verschieden H, D, F, C(=O)R³, CN, Si(R³)₃, N(R³)₂, P(=O)(R³)₂, S(=O)R³, S(=O)₂R³, eine geradkettige Alkyl-, oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch-R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F oder CN ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann, wobei zwei oder mehr Reste R² miteinander verknüpft sein können und einen aliphatischen oder heteroaliphatischen Ring bilden können;
R³ ist bei jedem Auftreten gleich oder verschieden H, D, F, C(=O)R⁴, CN, Si(R⁴)₃, N(R⁴)₂, P(=O)(R⁴)₂, S(=O)R⁴, S(=O)₂R⁴, eine geradkettige Alkyl-, oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch-R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können und wobei ein oder mehrere H-Atome in den oben genannten Gruppen durch D, F oder CN ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxy- oder Heteroaryloxygruppe mit 5 bis 30 aromatischen Ringatomen, die durch einen oder mehrere Reste R⁴ substituiert sein kann, wobei zwei oder mehr Reste R³ miteinander verknüpft sein können und einen Ring bilden können;
R⁴ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden;
n ist gleich 0 oder 1;
wobei mindestens eine Gruppe R¹ oder R² in der Verbindung der Formel (I) gewählt ist aus einem aromatischen oder heteroaromatischen Ringsystem mit 5 bis 40 aromatischen Ringatomen, das mit einem oder mehreren Resten R³ substituiert sein kann.

2. Elektronische Vorrichtung nach Anspruch 1, **dadurch gekennzeichnet, dass** mindestens eine Gruppe R¹ oder R² in der Verbindung der Formel (I) gewählt ist aus einer Gruppe enthaltend mindestens eine der folgenden Gruppen:
- Heteroarylgruppen mit 5 bis 20 aromatischen Ringatomen, die mindestens einen heteroaromatischen Fünfring mit zwei oder mehr Heteroatomen gewählt aus N, O und S enthalten;
- Heteroarylgruppen mit 6 bis 20 aromatischen Ringatomen, die mindestens einen heteroaromatischen Sechsring mit einem oder mehreren Heteroatomen gewählt aus N, O und S enthalten; und
- Carbazolgruppen.

3. Elektronische Vorrichtung nach Anspruch 1 oder 2, **dadurch gekennzeichnet, dass** mindestens eine Gruppe R¹ oder R² in der Verbindung der Formel (I) gewählt ist aus Gruppen der Formeln (Het-a) bis (Het-e) 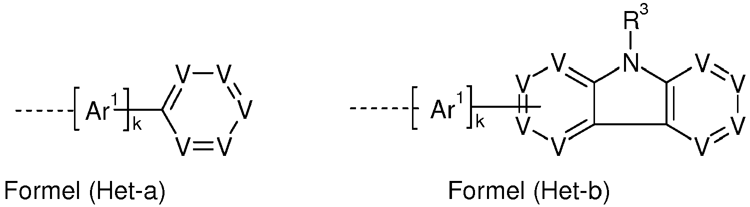 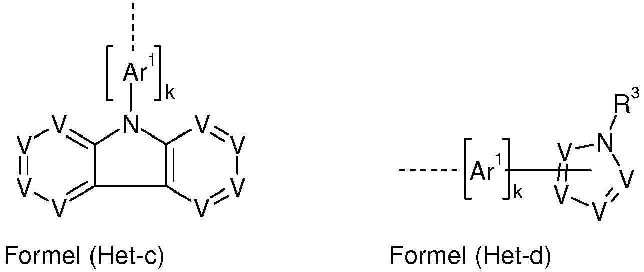 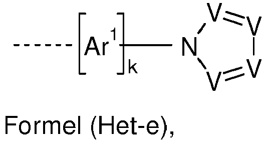 wobei
Ar¹ ein aromatisches oder heteroaromatisches Ringsystem mit 5 bis 20 aromatischen Ringatomen ist, das mit einem oder mehreren Resten R³ substituiert sein kann;
V bei jedem Auftreten gleich oder verschieden N oder CR³ ist;
k gleich 0 oder 1 ist;
die gestrichelte Linie die Bindung an den Rest der Verbindung bezeichnet;
und wobei in Formel (Het-a), (Het-d) und (Het-e) mindestens eine Gruppe V im Ring gleich N ist.

4. Elektronische Vorrichtung nach einem oder mehreren der Ansprüche 1 bis 3, **dadurch gekennzeichnet, dass** der Index n in Formel (I) gleich 1 ist.

5. Elektronische Vorrichtung nach einem oder mehreren der Ansprüche 1 bis 4, **dadurch gekennzeichnet, dass** alle Gruppen Z in Formel (I) gleich CR² sind.

6. Elektronische Vorrichtung nach einem oder mehreren der Ansprüche 1 bis 5, **dadurch gekennzeichnet, dass** die Gruppe W in Formel (I) gleich CR¹ ist.

7. Elektronische Vorrichtung nach einem oder mehreren der Ansprüche 1 bis 6, **dadurch gekennzeichnet, dass** die Gruppe E in Formel (I) bei jedem Auftreten gleich oder verschieden eine Einfachbindung, C=O, N(R¹), O, S, S=O oder S(=O)₂ ist, wobei nicht beide Gruppen E eine Einfachbindung sein dürfen.

8. Elektronische Vorrichtung nach einem oder mehreren der Ansprüche 1 bis 7, **dadurch gekennzeichnet, dass** sie gewählt ist aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs).

9. Elektronische Vorrichtung nach einem oder mehreren der Ansprüche 1 bis 8, **dadurch gekennzeichnet, dass** sie die Verbindung der Formel (I) in einer Elektronentransportschicht enthält, oder dass sie die Verbindung der Formel (I) in einer emittierenden Schicht als Matrixmaterial in Kombination mit einem oder mehreren Dotanden enthält.

## Claims

1. Electronic device comprising anode, cathode and at least one organic layer comprising at least one compound of the formula (I) 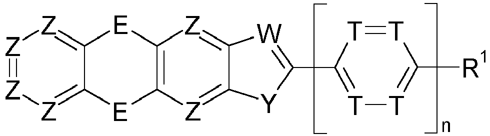 formula (I)
where:
E is selected on each occurrence, identically or differently, from a single bond, B(R¹), C=O, N(R¹), P(R¹), P(=O)R¹, O, S, S=O and S(=O)₂, where the groups E cannot both be a single bond;
T is on each occurrence, identically or differently, CR¹ or N;
W is CR¹ or N;
Y is N(R¹), O or S;
Z is on each occurrence, identically or differently, CR² or N;
R¹ is on each occurrence, identically or differently, H, D, F, C(=O)R³, CN, Si(R³)₃, N(R³)₂, P(=O)(R³)₂, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R³ and where one or more CH₂ groups in the above-mentioned groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂ and where one or more H atoms in the above-mentioned groups may be replaced by D, F or CN, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³;
R² is on each occurrence, identically or differently, H, D, F, C(=O)R³, CN, Si(R³)₃, N(R³)₂, P(=O)(R³)₂, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R³ and where one or more CH₂ groups in the above-mentioned groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂ and where one or more H atoms in the above-mentioned groups may be replaced by D, F or CN, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³, where two or more radicals R² may be linked to one another and may form an aliphatic or heteroaliphatic ring;
R³ is on each occurrence, identically or differently, H, D, F, C(=O)R⁴, CN, Si(R⁴)₃, N(R⁴)₂, P(=O)(R⁴)₂, S(=O)R⁴, S(=O)₂R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R⁴ and where one or more CH₂ groups in the above-mentioned groups may be replaced by -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO or SO₂ and where one or more H atoms in the above-mentioned groups may be replaced by D, F or CN, or an aromatic or heteroaromatic ring system having 5 to 30 aromatic ring atoms, which may in each case be substituted by one or more radicals R⁴, or an aryloxy or heteroaryloxy group having 5 to 30 aromatic ring atoms, which may be substituted by one or more radicals R⁴, where two or more radicals R³ may be linked to one another and may form a ring;
R⁴ is on each occurrence, identically or differently, H, D, F or an aliphatic, aromatic or heteroaromatic organic radical having 1 to 20 C atoms, in which, in addition, one or more H atoms may be replaced by D or F; two or more substituents R⁴ may be linked to one another and may form a ring;
n is equal to 0 or 1;
where at least one group R¹ or R² in the compound of the formula (I) is selected from an aromatic or heteroaromatic ring system having 5 to 40 aromatic ring atoms, which may be substituted by one or more radicals R³.

2. Electronic device according to Claim 1, **characterised in that** at least one group R¹ or R² in the compound of the formula (I) is selected from a group containing at least one of the following groups:
- heteroaryl groups having 5 to 20 aromatic ring atoms which contain at least one heteroaromatic five-membered ring having two or more heteroatoms selected from N, O and S;
- heteroaryl groups having 6 to 20 aromatic ring atoms which contain at least one heteroaromatic six-membered ring having one or more heteroatoms selected from N, O and S; and
- carbazole groups.

3. Electronic device according to Claim 1 or 2, **characterised in that** at least one group R¹ or R² in the compound of the formula (I) is selected from groups of the formulae (Het-a) to (Het-e) 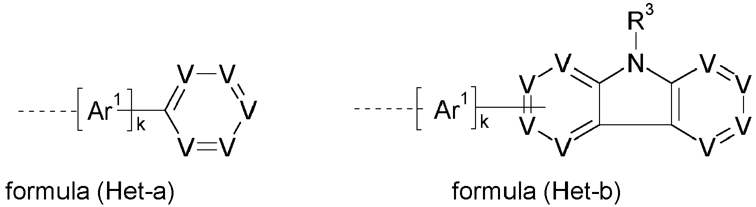 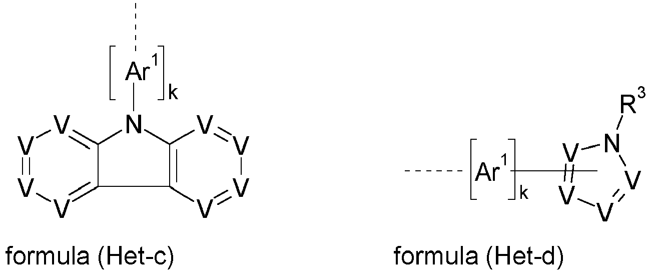 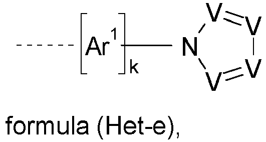 where
Ar¹ is an aromatic or heteroaromatic ring system having 5 to 20 aromatic ring atoms, which may be substituted by one or more radicals R³;
V is on each occurrence, identically or differently, N or CR³;
k is equal to 0 or 1;
the dashed line denotes the bond to the remainder of the compound;
and where at least one group V in the ring in formulae (Het-a), (Het-d) and (Het-e) is equal to N.

4. Electronic device according to one or more of Claims 1 to 3, **characterised in that** the index n in formula (I) is equal to 1.

5. Electronic device according to one or more of Claims 1 to 4, **characterised in that** all groups Z in formula (I) are equal to CR².

6. Electronic device according to one or more of Claims 1 to 5, **characterised in that** the group W in formula (I) is equal to CR¹.

7. Electronic device according to one or more of Claims 1 to 6, **characterised in that** the group E in formula (I) is on each occurrence, identically or differently, a single bond, C=O, N(R¹), O, S, S=O or S(=O)₂, where the groups E cannot both be a single bond.

8. Electronic device according to one or more of Claims 1 to 7, **characterised in that** it is selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs).

9. Electronic device according to one or more of Claims 1 to 8, **characterised in that** it comprises the compound of the formula (I) in an electron-transport layer, or **in that** it comprises the compound of the formula (I) in an emitting layer as matrix material in combination with one or more dopants.

## Revendications

1. Dispositif électronique comprenant une anode, une cathode et au moins une couche organique qui comprend au moins un composé de la formule (I) 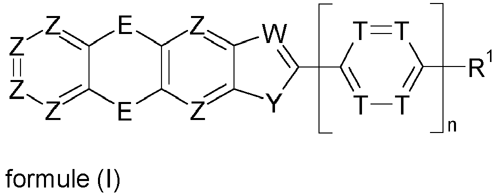 dans laquelle :
E est sélectionné pour chaque occurrence, de manière identique ou différente, parmi une liaison simple, B(R¹), C=O, N(R¹), P(R¹), P(=O)R¹, O, S, S=O et S(=O)₂, où les groupes E ne peuvent pas être tous les deux une liaison simple ;
T est pour chaque occurrence, de manière identique ou différente, CR¹ ou N ;
W est CR¹ ou N ;
Y est N(R¹), O ou S ;
Z est pour chaque occurrence, de manière identique ou différente, CR² ou N ;
R¹ est pour chaque occurrence, de manière identique ou différente, H, D, F, C(=O)R³, CN, Si(R³)₃, N(R³)₂, P(=O)(R³)₂, S(=O)R³, S(=O)₂R³, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=NR³, -C(=O)O-, -C(=O)NR³⁻, NR³, P(=O)(R³), -O-, -S-, SO ou SO₂ et où un ou plusieurs atome(s) de H dans les groupes qui ont été mentionnés ci-avant peut/peuvent être remplacé(s) par D, F ou CN, ou un système de cycle aromatique ou hétéroaromatique qui comporte de 5 à 30 atomes de cycle aromatique, lequel peut dans chaque cas être substitué par un radical ou par plusieurs radicaux R³, ou un groupe aryloxy ou hétéroaryloxy qui comporte de 5 à 30 atomes de cycle aromatique, lequel peut être substitué par un radical ou par plusieurs radicaux R³ ;
R² est pour chaque occurrence, de manière identique ou différente, H, D, F, C(=O)R³, CN, Si(R³)₃, N(R³)2, P(=O)(R³)₂, S(=O)R³, S(=O)₂R³, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=NR³, -C(=O)O-, -C(=O)NR³-, NR³, P(=O)(R³), -O-, -S-, SO ou SO₂ et où un ou plusieurs atome(s) de H dans les groupes qui ont été mentionnés ci-avant peut/peuvent être remplacé(s) par D, F ou CN, ou un système de cycle aromatique ou hétéroaromatique qui comporte de 5 à 30 atomes de cycle aromatique, lequel peut dans chaque cas être substitué par un radical ou par plusieurs radicaux R³, ou un groupe aryloxy ou hétéroaryloxy qui comporte de 5 à 30 atomes de cycle aromatique, lequel peut être substitué par un radical ou par plusieurs radicaux R³, où deux radicaux R² ou plus peuvent être liés l'un à l'autre ou les uns aux autres et peuvent former un cycle aliphatique ou hétéroaliphatique ;
R³ est pour chaque occurrence, de manière identique ou différente, H, D, F, C(=O)R⁴, CN, Si(R⁴)₃, N(R⁴)₂, P(=O)(R⁴)₂, S(=O)R⁴, S(=O)₂R⁴, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R⁴ et où un ou plusieurs groupe(s) CH₂ dans les groupes qui ont été mentionnés ci-avant peut/peuvent être remplacé(s) par -R⁴C=CR⁴-, -C≡C-, Si(R⁴)₂, C=O, C=NR⁴, -C(=O)O-, -C(=O)NR⁴- , NR⁴, P(=O)(R⁴), -O-, -S-, SO ou SO₂ et où un ou plusieurs atome(s) de H dans les groupes qui ont été mentionnés ci-avant peut/peuvent être remplacé(s) par D, F ou CN, ou un système de cycle aromatique ou hétéroaromatique qui comporte de 5 à 30 atomes de cycle aromatique, lequel peut dans chaque cas être substitué par un radical ou par plusieurs radicaux R⁴, ou un groupe aryloxy ou hétéroaryloxy qui comporte de 5 à 30 atomes de cycle aromatique, lequel peut être substitué par un radical ou par plusieurs radicaux R⁴, où deux radicaux R³ ou plus peuvent être liés l'un à l'autre ou les uns aux autres et peuvent former un cycle ;
R⁴ est pour chaque occurrence, de manière identique ou différente, H, D, F ou un radical organique aliphatique, aromatique ou hétéroaromatique qui comporte de 1 à 20 atome(s) de C, où, en outre, un ou plusieurs atome(s) de H peut/ peuvent être remplacé(s) par D ou par F ; deux substituants R⁴ ou plus peuvent être liés l'un à l'autre ou les uns aux autres et peuvent former un cycle ;
n est égal à 0 ou 1 ;
où au moins un groupe R¹ ou R² dans le composé de la formule (I) est sélectionné parmi un système de cycle aromatique ou hétéroaromatique qui comporte de 5 à 40 atomes de cycle aromatique, lequel peut être substitué par un radical ou par plusieurs radicaux R³.

2. Dispositif électronique selon la revendication 1, **caractérisé en ce qu'**au moins un groupe R¹ ou R² dans le composé de la formule (I) est sélectionné parmi un groupe qui contient au moins l'un des groupes qui suivent :
- des groupes hétéroaryle qui comportent de 5 à 20 atomes de cycle aromatique, lesquels contiennent au moins un cycle à cinq éléments hétéroaromatique qui comporte deux hétéroatomes ou plus qui sont sélectionnés parmi N, O et S ;
- des groupes hétéroaryle qui comportent de 6 à 20 atomes de cycle aromatique, lesquels contiennent au moins un cycle à six éléments aromatique qui comporte un ou plusieurs hétéroatome(s) qui est/sont sélectionné(s) parmi N, O et S ; et
- des groupes carbazole.

3. Dispositif électronique selon la revendication 1 ou 2, **caractérisé en ce qu'**au moins un groupe R¹ ou R² dans le composé de la formule (I) est sélectionné parmi les groupes des formules (Het-a) à (Het-e) : 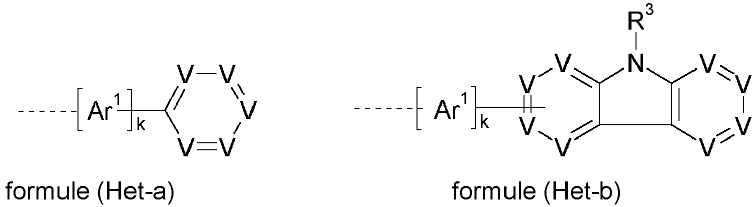 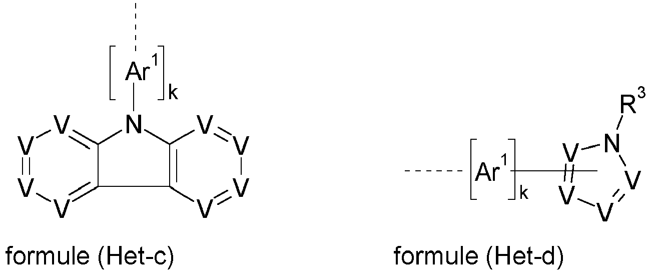 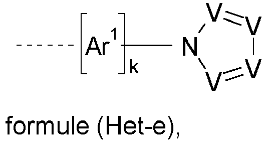 dans lesquelles :
Ar¹ est un système de cycle aromatique ou hétéroaromatique qui comporte de 5 à 20 atomes de cycle aromatique, lequel peut être substitué par un radical ou par plusieurs radicaux R³ ;
V est pour chaque occurrence, de manière identique ou différente, N ou CR³ ;
k est égal à 0 ou 1 ;
la ligne en pointillés représente la liaison sur le reste du composé ;
et où au moins un groupe V dans le cycle des formules (Het-a), (Het-d) et (Het-e) est égal à N.

4. Dispositif électronique selon une ou plusieurs des revendications 1 à 3, **caractérisé en ce que** l'indice n dans la formule (I) est égal à 1.

5. Dispositif électronique selon une ou plusieurs des revendications 1 à 4, **caractérisé en ce que** tous les groupes Z dans la formule (I) sont égaux à CR².

6. Dispositif électronique selon une ou plusieurs des revendications 1 à 5, **caractérisé en ce que** le groupe W dans la formule (I) est égal à CR¹.

7. Dispositif électronique selon une ou plusieurs des revendications 1 à 6, **caractérisé en ce que** le groupe E dans la formule (I) est pour chaque occurrence, de manière identique ou différente, une liaison simple, C=O, N(R¹), O, S, S=O ou S(=O)₂, où les groupes E ne peuvent pas être tous les deux une liaison simple.

8. Dispositif électronique selon une ou plusieurs des revendications 1 à 7, **caractérisé en ce qu'**il est sélectionné parmi le groupe qui est constitué par les circuits intégrés organiques (OIC), les transistors à effet de champ organiques (OFET), les transistors à film mince organiques (OTFT), les transistors à émission de lumière ou électroluminescents organiques (OLET), les cellules solaires organiques (OSC), les détecteurs optiques organiques, les photorécepteurs organiques, les dispositifs à extinction de champ organiques (OFQD), les cellules électrochimiques à émission de lumières ou électroluminescentes organiques (OLEC), les diodes laser organiques (O-laser) et les dispositifs électroluminescents organiques (OLED).

9. Dispositif électronique selon une ou plusieurs des revendications 1 à 8, **caractérisé en ce qu'**il comprend le composé de la formule (I) dans une couche de transport d'électrons, ou **en ce qu'**il comprend le composé de la formule (I) dans une couche d'émission en tant que matériau de matrice en combinaison avec un ou plusieurs dopant(s).
